# EUROPÄISCHE PATENTSCHRIFT

(11) **EP 1 810 713 B1**
(45) Veröffentlichungstag und Bekanntmachung des Hinweises auf die Patenterteilung: **24.11.2010**
(21) Anmeldenummer: 07001495.6
(22) Anmeldetag: 24.01.2007
(51) Int. Cl.: A61N 5/06

(54) **Bräunungsgerät mit Vorrichtung zum benutzerseitigen Handhaben eines Bräunungsgeräteoberteils**
Tanning device with user operation of the upper part of the tanning device
Appareil de bronzage comprenant un dispositif pour la manipulation d'une partie supérieure de l'appareil de bronzage

(30) Priorität: 24.01.2006 DE 102006003381
(43) Veröffentlichungstag der Anmeldung: 25.07.2007
(73) Patentinhaber: JK-Holding GmbH, 53578 Windhagen (DE)
(72) Erfinder: Kratz, Walter, 3620 Lanaken-Neerharen (BE)
(74) Vertreter: Koepe, Gerd L.

(56) Entgegenhaltungen:
- EP-A1- 1 529 552
- EP-A2- 0 388 944
- DE-A1- 2 550 327
- DE-A1- 3 303 795
- DE-A1- 3 708 820
- DE-A1- 19 602 018
- DE-A1- 19 808 953
- DE-A1- 19 836 825
- FR-B- 1 005 211
- US-A- 4 660 561
- US-B1- 6 461 376

## Beschreibung

Die vorliegende Erfindung betrifft eine Vorrichtung zum benutzerseitigen Handhaben des Oberteils eines Bräunungsgeräts, die vorzugsweise am Oberteil eines Bräunungsgeräts und noch mehr bevorzugt im Innern des Bräunungstunnels angebracht ist und ermöglicht, dass ein Benutzer des Bräunungsgeräts dieses im Liegen dadurch handhaben, beispielsweise öffnen oder schließen kann, dass er mittels der Vorrichtung das Oberteil des Bräunungsgeräts zu sich hin oder von sich weg schwenkt und dadurch die im Oberteil angeordneten, UV-Strahlung mit zumindest einer bräunenden Komponente emittierenden UV-Licht-Quellen in die korrekte, über dem Benutzer liegende Position zur Abgabe der UV-Strahlung bringt oder das Bräunungsgerät öffnet. In vorteilhaften Ausführungsformen der Erfindung können der Handhabungs-Vorrichtung weitere Funktionen zukommen. Die Erfindung betrifft auch ein eine derartige Handhabungs-Vorrichtung umfassendes Bräunungsgerät.

Bräunungsgeräte, mit denen heutzutage eine Bestrahlung des menschlichen Körpers mit UV-Strahlung umfassender Strahlung durchgeführt werden kann, bestehen regelmäßig aus einem statischen Unterteil, auf dem der Benutzer sitzenderweise oder liegenderweise auf einer Sitz- oder Liegefläche aus für UV-Strahlung zumindest partiell durchsichtigem Material, wie beispielsweise Acrylglas, Platz nimmt, unterhalb der die Strahlungsquellen für UV-Strahlung umfassende Strahlung, wie beispielsweise UV-Strahlung emittierende Röhren, und ein Großteil der Steuerungselektronik des gesamten Bräunungsgeräts angeordnet sind, und einem zur Erleichterung des Platznehmens auf dem Bräunungsgerät beweglichen, üblicherweise um eine Scharniergruppe an einer der Längsseiten des Bräunungsgeräts schwenkbaren, das statische Unterteil im geschlossenen Zustand im wesentlichen abdeckenden und einen (beispielsweise tunnelförmigen) Bräunungsraum bildenden Oberteil, in dem sich ebenfalls Strahlungsquellen für UV-Strahlung umfassende Strahlung, wie beispielsweise UV-Strahlung emittierende Röhren, oberhalb einer für UV-Strahlung wenigstens partiell durchsichtigen Schutzplatte, wie beispielsweise einer Acrylglas-Schutzplatte, angeordnet sind. In heute üblichen Bräunungsgeräten finden sich neben UV-Strahlung emittierenden Röhren für die Bestrahlung des gesamten Körpers des Benutzers häufig Anordnungen, mit denen bestimmte Bereiche des Körpers des Benutzers gezielt bestrahlt werden können, beispielsweise Hochdruck-Gesichtsbräuner für das Gesicht des Benutzers oder Schulterbräuner für die Schulter des Benutzers.

Das Platznehmen des Benutzers auf/in dem Bräunungsgerät ist umso bequemer möglich, je weiter sich das Oberteil vor dem Platznehmen öffnen lässt. Das Verschließen des Bräunungsgeräts, üblicherweise durch Schwenken des beweglichen Oberteils um die Scharniere, erfolgt entweder durch einen automatischen Mechanismus auf Knopfdruck, was den - mitunter aufwendigen - Einbau entsprechender Einrichtungen erfordert, oder manuell durch Herabziehen des Oberteils des Bräunungsgeräts durch den Benutzer während des Platznehmens auf der Sitz- oder Liegefläche oder bereits im Liegen, wie dies bereits in der Druckschrift DE-A 198 08 953 offenbart wurde. Ein der Bequemlichkeit dienender großer Öffnungswinkel erschwert jedoch das Schließen, insbesondere wenn dieses manuell erfolgen muß. Beim Platznehmen des Benutzers entstand häufig auch das Problem, dass das Oberteil schneller abgesenkt wird, als sich der Benutzer in die sitzende oder liegende Stellung begeben kann. Die Folge waren Kollisionen des Oberteils des Bräunungsgeräts, insbesondere seiner Kante, mit dem Körper, insbesondere mit dem Kopf, des Benutzers, was natürlich unerwünscht war. Zuverlässige Lösungen für ein manuelles Verschließen des Oberteils eines Bräunungsgeräts von innen durch den Benutzer, insbesondere im Liegen, waren bisher nicht verfügbar. Dies lag zum Teil auch daran, dass die relativ aufwendige Konstruktion des Oberteils eines Bräunungsgeräts das Anbringen einer Handhabungs-Vorrichtung wie z. B. eines Handgriffs im Innern des Bräunungstunnels nur schlecht erlaubte.

Die Druckschrift DE-A 198 36 825 beschreibt ein Bräunungsgerät mit einem standfesten Unterteil und einem mit dem Unterteil über ein längsseitiges Scharnier verbundenes und schwenkbares Oberteil. Das Bräunungsgerät weist in seinem Innern eine oder mehrere Informationseinrichtungen und/oder eine oder mehrere Bildschirmeinrichtungen auf, die mittels einer an der Informations- bzw. Bildschirmeinrichtung angebrachten Bedieneinrichtung bedient werden können.

In der Druckschrift US-A 6,461,376 wird ein oder werden mehrere Handgriffe im Oberteil eines Bräunungsgeräts beschrieben, in die die Hand oder das Handgelenk eines Benutzers eingelegt und damit dessen Arm bzw. Arme für die Dauer des Bräunungsvorgangs bequem angehoben werden kann/können, um eine Bräunung auch im Bereich der Unterarme bzw. unter den Armen erreichen zu können.

Ein weiteres Bräunungsgerät ist aus der Druckschrift DT 25 50 327 A1 bekannt.

Es bestand daher ein Bedarf nach einer einfachen Vorrichtung, mit der das Oberteil eines Bräunungsgeräts manuell von der vollständig offenen in die vollständig "geschlossene" Position gebracht werden kann, wenn der Benutzer bereits auf der Sitzfläche bzw. Liegefläche Platz genommen hat, bzw. das Bräunungsgerät durch "Aufschwenken" des Oberteils manuell geöffnet werden kann, wenn der Bräunungsvorgang beendet ist. Dieser Bedarf wird durch die Vorrichtung der vorliegenden Erfindung zum manuellen Handhaben eines Bräunungsgeräte-Oberteils geregelt.

Weiter wurde in einer Ausführungsform der Erfindung, die besonders vorteilhaft ist, gefunden, dass der Benutzer im wesentlichen gleichzeitig mit der Tätigkeit des Verschließens des Bräunungsgeräts mittels der erfindungsgemäßen Handhabungs-Vorrichtung bestimmte Steuerungstätigkeiten für den Start des Bräunungsgeräts und den Verlauf des Bräunungsvorgangs dann durchführen kann, wenn die Handhabungs-Vorrichtung über die entsprechenden Steuerungselemente verfügt und die Überwachung des Steuerungsvorgangs durch den Benutzer an einer Stelle erfolgen kann, die im Bereich der optischen bzw. akustischen Wahrnehmung des Benutzers während des Vorgangs des Verschließens und des anschließenden Bräunens liegt.

Die Erfindung betrifft daher ein Bräunungsgerät, das umfasst:
- ein standfestes Unterteil mit Kopfende und Fußende und zwei Längsseiten, enthaltend elektrische Aggregate, Schalt-Einrichtungen und Verbindungs-Einrichtungen; und
- ein entweder an einer der Längsseiten des Unterteils über Scharniere oder Gelenke angebrachtes, über die Scharniere oder Gelenke von dem Unterteil weg und auf das Unterteil hin zur Bildung eines Bräunungsraumes klappbares oder schwenkbares oder an einer separaten Aufhängung von dem Unterteil weg hebbares und auf das Unterteil hin zur Bildung eines Bräunungsraumes senkbares Oberteil mit Kopfende und Fußende und zwei Längsseiten; wobei

- das Unterteil eine zumindest für bräunende UV-Strahlung durchlässige Liegefläche für den Benutzer und unterhalb der Liegefläche mindestens einen Satz in Längsrichtung des Bräunungsgeräts angeordneter Strahler und gegebenenfalls noch weitere Strahler zur Abgabe von Strahlung mit zumindest einem Anteil an bräunender UV-Strahlung in Richtung auf das Oberteil und/oder zumindest einen Teil des Körpers des Benutzers gerichtet mit den elektrischen Aggregaten, Schalt-Einrichtungen und/oder Verbindungs-Einrichtungen direkt oder indirekt verbunden aufweist; und
- das Oberteil eine zumindest für bräunende UV-Strahlung durchlässige Schutzfläche für den Benutzer und oberhalb der Schutzfläche mindestens einen Satz in Längsrichtung des Bräunungsgeräts angeordneter Strahler und gegebenenfalls noch weitere Strahler zur Abgabe von Strahlung mit zumindest einem Anteil an bräunender UV-Strahlung in Richtung auf das Unterteil und/oder zumindest einen Teil des Körpers des Benutzers gerichtet aufweist.

Das so ausgebildete Bräunungsgerät umfasst nun erfindungsgemäß an seinem Oberteil
- eine durch den Benutzer manuell bedienbare Handhabungs-Vorrichtung für das Bräunungsgeräte-Oberteil, wobei die Handhabungs-Vorrichtung Einrichtungen zum Anwählen und Steuern aller Funktionen im Bräunungsgerät aufweist.

In einer bevorzugten Ausführungsform umfasst das vorstehend beschriebene Bräunungsgerät
- im Bereich der Schutzfläche des Oberteils, im Bereich der Sicht des Benutzers, eine bogenförmige Befestigungseinrichtung mit einem sich in Richtung auf das Kopfende erstreckenden Befestigungsfortsatz, an dem die durch den Benutzer manuell bedienbare Handhabungs-Vorrichtung für das Bräunungsgeräte-Oberteil konkavseitig angebracht ist.

Die Erfindung betrifft weiter eine Handhabungs-Vorrichtung zum manuellen Verschließen und Öffnen des Oberteils eines Bräunungsgeräts, das umfasst:
- ein standfestes Unterteil mit Kopfende und Fußende und zwei Längsseiten, enthaltend elektrische Aggregate, Schalt-Einrichtungen und Verbindungs-Einrichtungen; und
- ein entweder an einer der Längsseiten des Unterteils über Scharniere oder Gelenke angebrachtes, über die Scharniere oder Gelenke von dem Unterteil weg und auf das Unterteil hin zur Bildung eines Bräunungsraumes klappbares oder schwenkbares oder an einer separaten Aufhängung von dem Unterteil weg hebbares und auf das Unterteil hin zur Bildung eines Bräunungsraumes senkbares Oberteil mit Kopfende und Fußende und zwei Längsseiten; wobei
- das Unterteil eine zumindest für bräunende UV-Strahlung durchlässige Liegefläche für den Benutzer und unterhalb der Liegefläche mindestens einen Satz in Längsrichtung des Bräunungsgeräts angeordneter Strahler und gegebenenfalls noch weitere Strahler zur Abgabe von Strahlung mit zumindest einem Anteil an bräunender UV-Strahlung in Richtung auf das Oberteil und/oder zumindest einen Teil des Körpers des Benutzers gerichtet mit den elektrischen Aggregaten, Schalt-Einrichtungen und/oder Verbindungs-Einrichtungen direkt oder indirekt verbunden aufweist; und
- das Oberteil eine zumindest für bräunende UV-Strahlung durchlässige Schutzfläche für den Benutzer und oberhalb der Schutzfläche mindestens einen Satz in Längsrichtung des Bräunungsgeräts angeordneter Strahler und gegebenenfalls noch weitere Strahler zur Abgabe von Strahlung mit zumindest einem Anteil an bräunender UV-Strahlung in Richtung auf das Unterteil und/oder zumindest einen Teil des Körpers des Benutzers gerichtet aufweist; wobei
- die Handhabungs-Vorrichtung am Oberteil des Bräunungsgeräts an einer für den Benutzer erreichbaren Stelle angebracht ist und Einrichtungen zum Anwählen und Steuern aller Funktionen im Bräunungsgerät aufweist.

In einer bevorzugten Ausführungsform weist das vorstehend beschriebene Bräunungsgerät an seinem Oberteil im Bereich der Schutzfläche im Bereich der Sicht des Benutzers eine bogenförmige Befestigungseinrichtung mit einem sich in Richtung auf das Kopfende erstreckenden Befestigungsfortsatz auf, an dem die durch den Benutzer manuell bedienbare Handhabungs-Vorrichtung konkavseitig angebracht ist.

Die Erfindung wird nachfolgend unter Bezugnahme auf die Figuren näher erläutert. Die Figuren - wie auch die nachfolgende detaillierte Beschreibung - betreffen bevorzugte Ausführungsformen der Erfindung; es ist jedoch nicht beabsichtigt, die Erfindung auf die in den Figuren gezeigten oder in der nachfolgenden Beschreibung als bevorzugt gekennzeichneten Ausführungsformen zu beschränken, die lediglich der Erläuterung der Erfindung dienen.

Es zeigen:

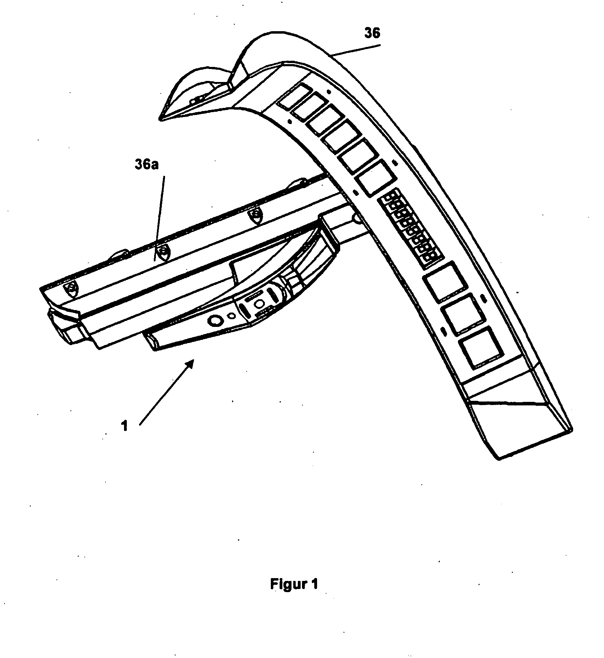

- Figur 1 eine Darstellung einer bevorzugt an einer bogenförmigen Befestigungsvorrichtung mit einem Befestigungsfortsatz befestigten Handhabungs-Vorrichtung in perspektivischer Ansicht;
- Figuren 2 A bis 2 E verschiedene Ausführungsformen bestimmter Handhabungs-Vorrichtungen;
- Figuren 3 A bis 3 C jeweils eine perspektivische Ansicht, eine Ansicht von unten und eine seitliche Ansicht einer Handhabungs-Vorrichtung an der Befestigungsvorrichtung; und
- Figur 4 ein herkömmliches Bräunungsgerät ohne Handhabungs-Vorrichtung.

Unter dem Begriff "Bräunungsgerät" werden in der nachfolgenden Beschreibung und in den Patentansprüchen Vorrichtungen verstanden, mit denen der Körper eines Benutzers, üblicherweise eines Menschen, mittels UV-Strahlung, die zumindest bräunende UV-Strahlung enthält, wenn nicht sogar weitgehend oder gar ausschließlich aus bräunender UV-Strahlung besteht, gebräunt wird. Dieser seit langer Zeit bekannte Vorgang besteht in einem Einstrahlen von Strahlung, vorzugsweise UV-Strahlung, noch weiter bevorzugt UV-Strahlung mit zumindest Anteilen von bräunender UV-Strahlung, die UV-A- und UV-B-Strahlung an sich bekannter Wellenlängen- und Intensitäts-Zusammensetzung umfasst, die von geeigneten Strahlungsquellen emittiert wird, beispielsweise von geeignet beaufschlagten UV-Röhren oder UV-Strahlern (NiederdruckStrahler, Hochdruck-Strahler) oder UV-Strahlung emittierende LED's, auf den menschlichen Körper oder zumindest Teile davon. Unter Bräunungsgeräte fallen solche Geräte, die der Bestrahlung des gesamten Körpers eines Benutzers dienen, und solche, die der Bestrahlung von Teilen des menschlichen Körpers dienen (beispielsweise Gesichtsbräuner oder Oberkörper-Bräuner), und auch solche, die der Bestrahlung des gesamten Körpers eines Benutzers dienen und zusätzlich Einrichtungen (wie beispielsweise zusätzliche Brenner und/oder Röhren und/oder LED's) aufweisen, mit denen gleichzeitig mit der Bestrahlung des gesamten Körpers eines Benutzers einzelne Partien des Körpers (z. B. das Gesicht, die Schultern, die Flanken) zusätzlich mit UV-Strahlung bestrahlt werden können. Besonders bevorzugte Bräunungsgeräte gemäß der Erfindung sind Ganzkörperbräuner, und noch weiter bevorzugt sind Ganzkörperbräuner, die zusätzliche Einrichtungen zur gezielten Bestrahlung bestimmter Körper-Partien aufweisen, beispielsweise zur Bestrahlung des Gesichts und/oder der Schultern.

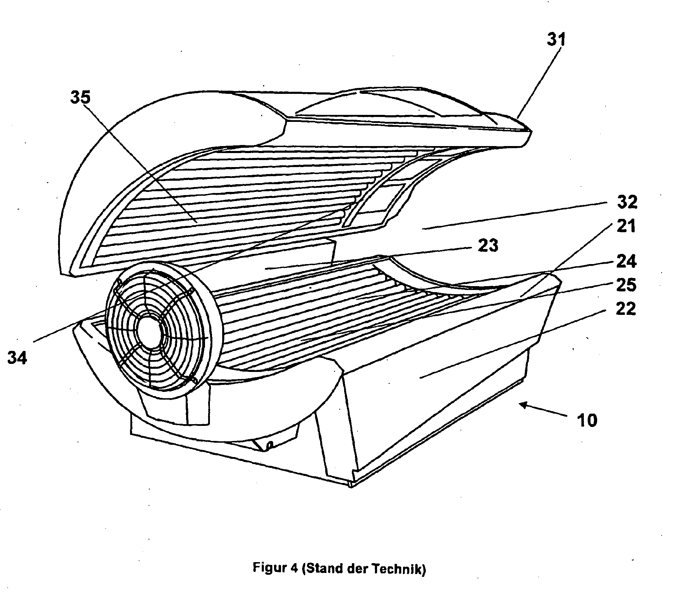

Bräunungsgeräte der vorstehend beschriebenen Art sind aus dem Stand der Technik bekannt und werden darin im Detail beschrieben. Bezug genommen werden kann beispielsweise auf eine detaillierte Beschreibung in der älteren Patentanmeldung Nr. DE 10 2005 030 388.9, deren den grundsätzlichen Aufbau eines Bräunungsgeräts betreffender Inhalt durch die Inbezugnahme in die vorliegende Anmeldung übernommen wird. Wie aus Figur 4 ersichtlich ist, die ein übliches, für das Bräunen des Benutzers im Liegen vorgesehenes Bräunungsgerät 10 zeigt (die Erfindung ist jedoch nicht auf diese beschränkt, sondern kann prinzipiell auch für Geräte vorgesehen werden, in/auf denen sich ein Benutzer im Sitzen oder in halb liegender Position bräunt), bestehen solche gattungsgemäßen Bräunungsgeräte 10 üblicherweise aus einem standfesten Unterteil 21, das neben einem Teil der UV-Strahlungsquellen die für den Betrieb des Geräts erforderlichen elektrischen Aggregate, Schalt-Einrichtungen und Verbindungseinrichtungen und gegebenenfalls noch weitere Einrichtungen wie beispielsweise eine Kühlung oder eine Belüftung oder Einrichtungen für Multimedia-Versorgung aufweist, und einem beweglichen Oberteil 31. Letzteres wird zum bequemen Platznehmen des Benutzers auf/in dem Bräunungsgerät 10 mit geeigneten Einrichtungen zumindest teilweise von dem standfesten Unterteil 21 entfernt, beispielsweise um Gelenke oder Scharniere 23, die meist (jedoch nicht beschränkend) eine der Längsseiten 22 des Unterteils 21 mit einer der Längsseiten des Oberteils 31 beweglich verbinden, in eine geöffnete Position geklappt oder geschwenkt, und wird nach dem Platznehmen des Benutzers wieder in die Ursprungs-Position zurückgeführt, wodurch sich um den Benutzer ein (beispielsweise) tunnelförmiger Bräunungsraum 32 bildet. Alternativ kann auch das Oberteil 31 an einer separaten Aufhängung von dem Unterteil 21 weg gehoben oder geschwenkt werden und kann nach Platznehmen des Benutzers auf dem Unterteil wieder in Richtung auf des Unterteil hin gesenkt oder geschwenkt werden. Derselbe Vorgang läuft nach Beendigung des Bräunungsvorgangs in umgekehrter Reihenfolge ab.

Zum Liegen des Benutzers des Bräunungsgeräts während des Bräunungsvorgangs weist das standfeste Unterteil 21 eine zumindest für bräunende UV-Strahlung durchlässige, meist (nicht jedoch beschränkend) weitgehend transparente Liegefläche 24 auf. Diese besteht aus einem zumindest für die bräunende UV-Strahlung durchlässigen Material. Dieses kann jedes dem Fachmann auf diesem Gebiet bekannte, für UV-Strahlung weitgehend transparente Material sein. Dieses Material hat nicht nur die erforderliche Transparenz für die UV-Anteile der Strahlung, sondern weist darüber hinaus auch weitere vorteilhafte Eigenschaften auf, wie beispielsweise eine ausreichende Stabilität und Flexibilität für das Tragen der zu bestrahlenden Person und eine hervorragende Stabilität gegen die Einwirkung reinigender und desinfizierender Substanzen, die nach dem Bräunungsvorgang angewendet werden müssen. Mit Vorteil wird ein Acryl-Polymer verwendet.

Unterhalb der Liegefläche 24 ist - bevorzugt in Längsrichtung des Bräunungsgeräts - mindestens ein Satz von Strahlungsquellen 25 für UV-Strahlung, insbesondere für bräunende UV-Strahlung, besonders bevorzugt bräunende UV-Strahlung emittierende Strahler-Röhren 25, denkbarerweise jedoch auch UV-Strahlung emittierende LED's, angeordnet. Die Strahlung wird bestimmungsgemäß in Richtung auf das Oberteil 31 des Bräunungsgeräts und damit auf den auf der Liegefläche 24 liegenden Benutzer des Bräunungsgeräts - oder zumindest auf einen Teil seines Körpers oder mehrere Teile seines Körpers - gerichtet. Gegebenenfalls können neben den genannten Strahlerröhren noch eine oder mehrere weitere Strahlungsquellen im Unterteil 21 des Bräunungsgeräts angeordnet sein, die für eine gezielte Bräunung bestimmter Bereiche des Körpers des Benutzers sorgen.

Das wie oben beschrieben mit dem standfesten Unterteil 21 verbundene und mit diesem zusammenwirkende Oberteil 31 des Bräunungsgerät weist eine zumindest für bräunende UV-Strahlung im wesentlichen durchlässige Schutzfläche 34 für den Benutzer auf, die zwischen dem Benutzer und den elektrisch betriebenen Aggregaten des Oberteils angeordnet ist. Diese Schutzfläche kann aus jedem beliebigen, dem Fachmann aufgrund seiner Fachkenntnis bekannten, zumindest für bräunende UV-Strahlung im wesentlichen durchlässigen und hinreichend stabilen Material bestehen. Bevorzugt wird für die Schutzfläche 34 ein Acryl-Polymer verwenden, wie es auch bei der Liegefläche 24 des Unterteils Anwendung findet. Die Schutzfläche 34 ist in einer bevorzugten Ausführungsform so geformt, dass sich über dem unter der Schutzfläche liegenden Benutzer des Bräunungsgeräts ein (beispielsweise, aber nicht beschränkend) tunnelförmiger Bräunungsraum 32 bildet, wobei besonders bevorzugt der höchste Teil des Tunnels ungefähr in der Mitte des Oberteils 31 liegt.

Oberhalb der (vorzugsweise einen tunnelförmigen Bräunungsraum 32 bildenden) Schutzfläche 34 ist mindestens ein Satz von - vorzugsweise in Längsrichtung des Bräunungsgeräts angeordneten - Strahlungsquellen 35 für UV-Strahlung, insbesondere für bräunende UV-Strahlung, angeordnet. Die Anordnung der UV-Strahlungsquellen 35 im Oberteil 31 des Bräunungsgeräts berücksichtigt die bevorzugte (aus Sicht der UV-Strahlungsquellen 35) konvexe Ausformung der Schutzfläche 34. Die UV-Strahlungsquellen 35 sind in der Mehrzahl der Fälle bräunende UV-Strahlung emittierende Strahler-Röhren 35, können jedoch auch andere, dem Fachmann geläufige UV-Strahlungsquellen wie beispielsweise UV-Strahlung emittierende LED's sein. Die Strahlung wird bestimmungsgemäß in Richtung auf das Unterteil 21 des Bräunungsgeräts und damit auf den auf der Liegefläche 24 liegenden Benutzer des Bräunungsgeräts - oder zumindest auf einen Teil seines Körpers oder mehrere Teile seines Körpers - gerichtet. Gegebenenfalls können neben den genannten Strahlerröhren 35 noch eine oder mehrere weitere Strahlungsquellen im Oberteil 31 des Bräunungsgeräts angeordnet sein, die speziell auf bestimmte Teile des Körpers des Benutzers wie z. B. auf das Gesicht des Benutzers gerichtet sind und für deren gezielte Bräunung sorgen.

Ein derartiges, an sich bekanntes, im Zusammenhang mit Figur 4 beschriebenes, gegebenenfalls weitere für das Bestrahlen des Körpers eines Benutzers nützliche bzw. sinnvolle Einrichtungen umfassendes Bräunungsgerät wird nun weiter im Zusammenhang mit der vorliegenden Erfindung unter Bezugnahme auf die Figuren beschrieben.

Erfindungsgemäß umfasst das so ausgebildete Bräunungsgerät an seinem Oberteil eine durch den Benutzer manuell bedienbare Handhabungs-Vorrichtung 1 für das Bräunungsgeräte-Oberteil. Die Handhabungs-Vorrichtung kann von beliebiger Art sein, die es gestattet, das Oberteil 31 durch den Benutzer manuell handhaben, insbesondere öffnen und schließen, zu lassen, bevorzugterweise das Oberteil 31 in einer liegenden Position auf dem Unterteil 21 öffnen und schließen zu lassen, ohne dass dies eines überstarken physischen Aufwandes oder unbequemer Verrenkungen bedarf. Weiter sollte die Handhabungs-Vorrichtung 1 das Ergebnis des Bräunungsvorgangs nicht beeinflussen, beispielsweise dadurch, dass sie an einer Stelle angebracht ist, die im Strahlengang der auf den Körper des Benutzers eingestrahlten UV-Strahlung liegt. Beispielhaft kommen infrage ein Handgriff am Oberteil 31 oder eine Griffmulde an geeigneter Stelle im Oberteil 31, beispielsweise in der Schutzfläche 34. Der Ort am Oberteil 31 des Bräunungsgeräts, an dem die Handhabungs-Vorrichtung 1 angebracht ist, ist nicht in irgendeiner Weise beschränkt, solange er die Handhabung des Oberteils 31 des Bräunungsgeräts durch den Benutzer ohne Probleme erlaubt und den Bräunungsvorgang nicht behindert. Vorzugsweise ist die Handhabungs-Vorrichtung 1 in dem Bereich des Oberteils 31 des Bräunungsgeräts angebracht, den der Benutzer im Liegen leicht erreichen und manuell bedienen kann, beispielsweise im Innenbereich des (vorzugsweise tunnelförmigen) Bräunungsraums 32, ohne jedoch die Erfindung hierauf zu beschränken.

In einer bevorzugten Ausführungsform der Erfindung weist das Bräunungsgerät im Bereich der Schutzfläche 34 des Oberteils 31 eine bogenförmige Befestigungseinrichtung 36 auf. Deren genaue Anordnung (entlang der Längsrichtung des Bräunungsgeräts) ist nicht beschränkt, jedoch liegt die bogenförmige Befestigungseinrichtung 36 aus Gründen, die sich aus der nachfolgenden Beschreibung ergeben, vorzugsweise im Bereich der Sicht des Benutzers, der auf der Liegefläche 24 liegt. Unter "bogenförmig" wird im Rahmen der vorliegenden Erfindung verstanden, dass die Befestigungseinrichtung 36 dem (aus Sicht des auf der Liegefläche 24 liegenden Benutzers) von links nach rechts konkaven Verlauf des Oberteils 31 des Bräunungsgeräts folgt und die höchste Stelle des "Bogens" der Befestigungseinrichtung 36 quasi in der Mitte im Bereich der höchsten Stelle des tunnelförmigen Bräunungsraums 32 bzw. der gebogenen Schutzfläche 34 liegt. Dabei muß "in der Mitte" nicht zwangsläufig exakt die Mitte bedeuten, sondern kann von der exakten räumlichen Mitte gegebenenfalls abweichen.

In dieser Ausführungsform ist auf der konkaven Seite der Befestigungseinrichtung 36, also "im Innern" des Bogens, an der (aus Sicht des auf der Liegefläche 24 des Unterteils 21 liegenden Benutzers) von links nach rechts laufenden Befestigungseinrichtung 36 ein Befestigungsfortsatz 36 a angebracht, der sich - wiederum aus Sicht des auf der Liegefläche liegenden Benutzers - in Richtung auf das Kopfende des Bräunungsgeräts erstreckt. Die Befestigungseinrichtung 36 und/oder der Befestigungsfortsatz 36 a bestehen aus einem stabilen Material wie beispielsweise aus einem Metall oder einem starren, gegebenenfalls verstärkten Kunststoff bzw. Kunststoff-Verbundmaterial. Es sind für diesen Zweck beliebige, dem Fachmann für Stabilisierungs- und Befestigungszwecke bekannte Materialien geeignet. Für die Auswahl leitend ist ein geringes Gewicht des Materials bei hoher Stabilität für die Verankerung im Oberteil 31 des Bräunungsgeräts und für die Befestigung der nachfolgend beschriebenen Handhabungs-Vorrichtung 1. Besonders bevorzugt verwendet wird für die Befestigungseinrichtung 36 und/oder den Befestigungsfortsatz 36 a ein Metall, ohne jedoch die Erfindung hierauf zu beschränken.

In der vorgenannten bevorzugten Ausführungsform umfasst das Bräunungsgerät eine durch den Benutzer mit der Hand bedienbare Handhabungs-Vorrichtung 1, die konkavseitig an dem Befestigungsfortsatz 36 a angebracht ist und damit vom oberen Teil des (beispielsweise tunnelförmigen) Bräunungsraums 32 des Oberteils 31 in Richtung auf den auf der Liegefläche 24 des Unterteils 21 liegenden Benutzer absteht. Das Vorsehen einer solchen Handhabungs-Vorrichtung 1 weist den wesentlichen Vorteil gegenüber dem Stand der Technik auf, dass der Benutzer auf der Liegefläche 24 des Unterteils 21 liegend das Oberteil 31 des Bräunungsgeräts manuell "schließen" und damit die im Oberteil 31 angeordneten UV-Strahlungsquellen 35 in die für den Bräunungsvorgang erforderliche Position über sich bringen - oder auch öffnen - kann. Die Handhabungs-Vorrichtung 1 stellt also ein einfaches und leicht zu verwirklichendes Mittel dar, um dem Benutzer zu ermöglichen, das Bräunungsgerät im Liegen manuell zu schließen und damit für den Beginn des Bräunungsvorgangs vorzubereiten oder zu öffnen und damit den Bräunungsvorgang zu beenden.

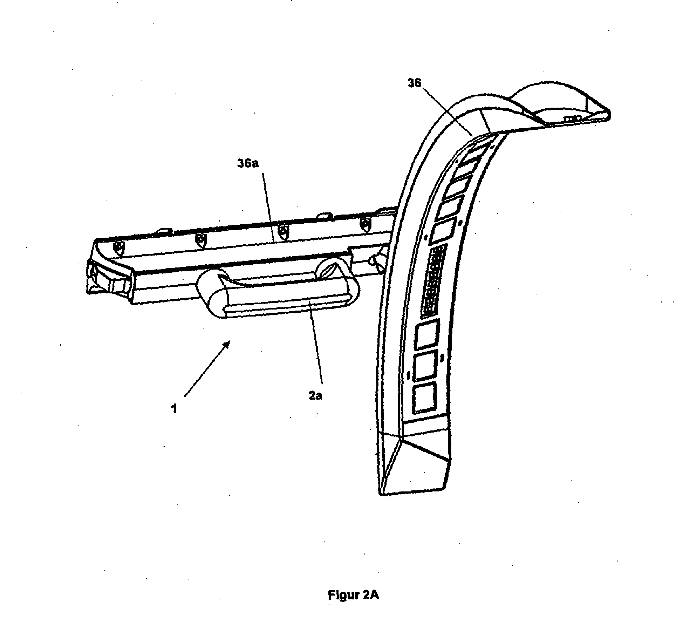

Die erfindungsgemäße, bevorzugt, aber nicht zwingend an dem Befestigungsfortsatz 36 a angebrachte und damit in Reichweite des Benutzers liegende Handhabungs-Vorrichtung 1 kann jede für den Fachmann denkbare Griff-Vorrichtung sein, ohne dass dies erfindungsgemäß einer Beschränkung unterworfen ist. In einer bevorzugten Ausführungsform der Erfindung ist die Handhabungs-Vorrichtung 1 eine U-förmige Griff-Vorrichtung 2 a, wie sie sich aus Figur 2 A ergibt. Eine derartige U-förmige Griff-Vorrichtung 2 a ist mit zwei Enden an dem Befestigungsfortsatz 36 a befestigt und erlaubt ein sicheres Ergreifen durch den Benutzer beim Vorgang des manuellen Schließens und Öffnens des Oberteils 31 des Bräunungsgeräts. Aus ergonomischen Gründen ist es weiter bevorzugt, dass die U-förmige Griff-Vorrichtung 2 a in Längsrichtung des Bräunungsraums 32 angeordnet ist.

In einer weiteren, erfindungsgemäß ebenfalls bevorzugten und daher mit Vorteil verwirklichten Ausführungsform ist die Handhabungs-Vorrichtung 1 ein offener

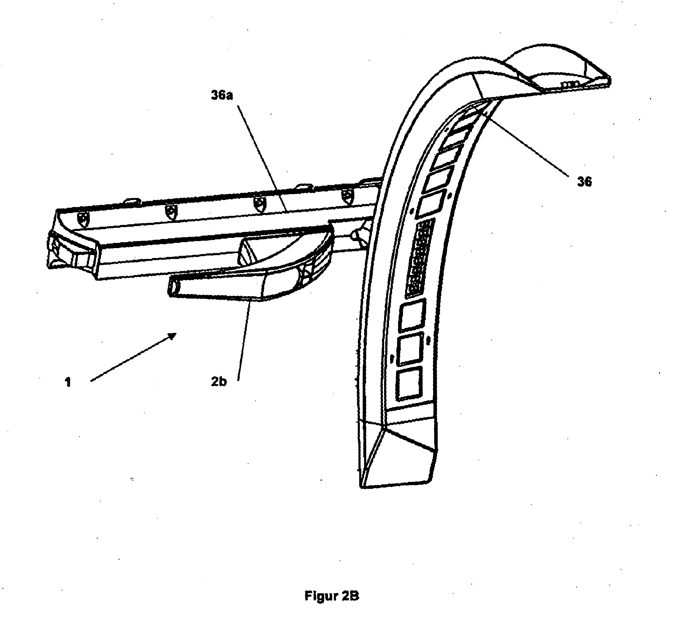

Griff 2 b, wie er sich aus Figur 2 B ergibt. Unter "offener Griff' wird dabei erfindungsgemäß eine Handhabungs-Vorrichtung 1 verstanden, deren einer Teil an dem Befestigungsfortsatz 36 a angebracht bzw. befestigt ist und deren anderer Teil von dem Befestigungsfortsatz 36 a beabstandet ist. Noch mehr bevorzugt ist aus ergonomischen Gründen, dass der offene Griff 2 b in Längsrichtung des Bräunungsraums 32 angeordnet ist, und noch weiter bevorzugt in der Weise, dass das offene Ende - wie aus Figur 2 B ersichtlich - zum Fußende des Bräunungsraums 32 hin angeordnet ist. Dies ist deswegen besonders vorteilhaft, weil sich dieser mit dem offenen Ende zum Fußende des Bräunungsraums 32 hin angeordnete Griff vom Benutzer im Liegen gut ergreifen und zum manuellen Schließen oder Öffnen des Oberteils 31 des Bräunungsgeräts ergreifen lässt.

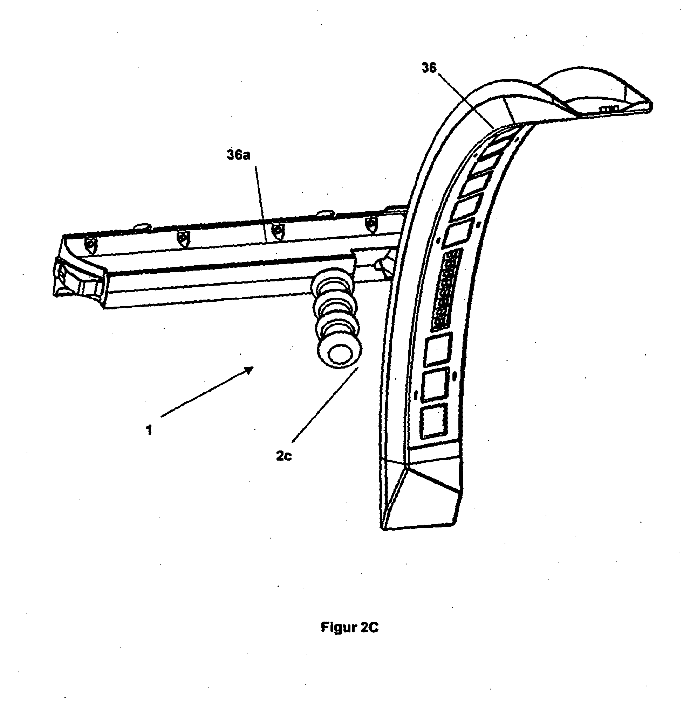

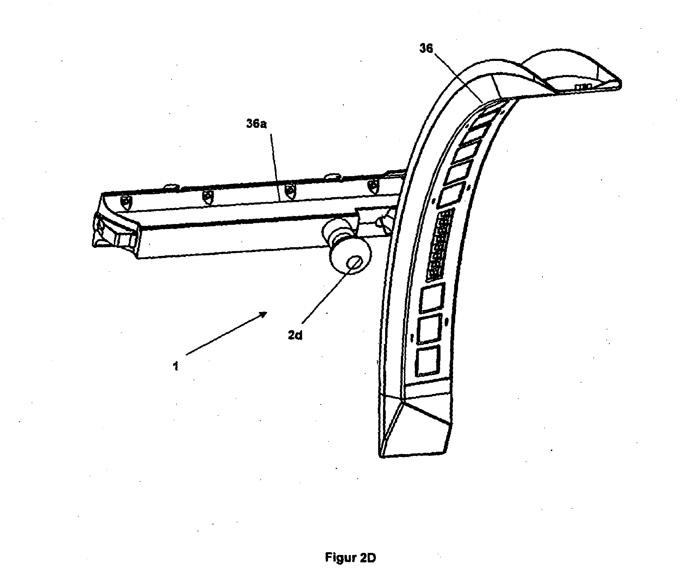

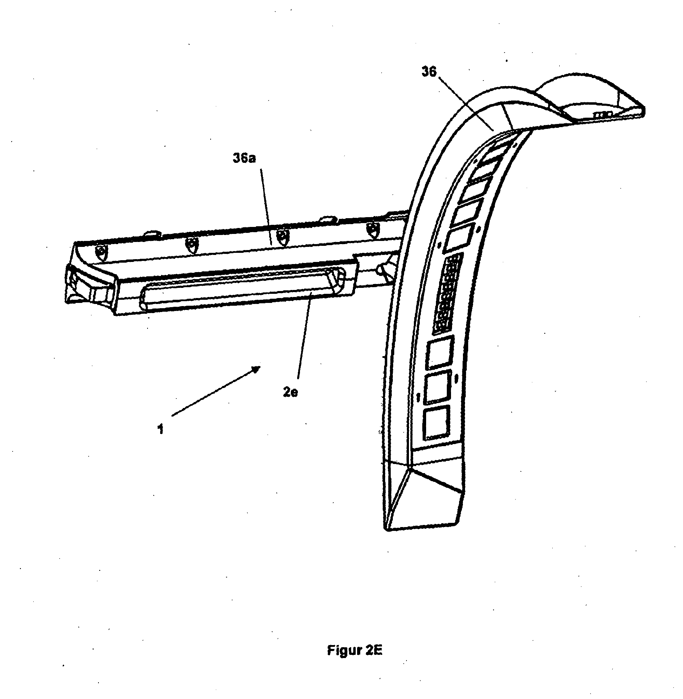

In weiteren bevorzugten Ausführungsformen der Erfindung kann die Handhabungs-Vorrichtung 1 auch ein stabartiger Griff 2 c oder ein knaufartiger Griff 2 d sein. Solche Griffe sind aus Figur 2 C und 2 D ersichtlich. Sie sind an dem Befestigungsfortsatz 36 a befestigt und stehen von diesem in Richtung auf den auf der Liegefläche 24 liegenden Benutzer des Bräunungsgeräts hervor, so dass sie bequem von diesem im Liegen ergriffen werden können, um das Oberteil 31 des Bräunungsgeräts manuell zu schließen oder zu öffnen. Alternativ kann die Handhabungs-Vorrichtung 1 auch eine Griffmulde 2 e sein, wie sie - wie in Figur 2 E gezeigt - im Oberteil 31 in Greifweite des Benutzers angebracht ist, beispielsweise in die Schutzfläche 34 im Oberteil 31 eingelassen ist.

Grundsätzlich unterliegt der Fachmann in der Wahl des Orts, an dem die Handhabungs-Vorrichtung 1 an dem Befestigungsfortsatz 36 a angebracht ist, keinen Beschränkungen, und es kann jeder beliebige Befestigungsort gewählt werden. In weiter bevorzugten Ausführungsformen sieht die Erfindung jedoch für die Befestigung der Griff-Vorrichtung 1 - unabhängig davon, in welcher Form sie ausgeprägt ist - zumindest zwei alternative bevorzugte Befestigungsmöglichkeiten vor. Im einen bevorzugten Fall ist die Griff-Vorrichtung 1 an einem an der höchsten Stelle der bogenförmigen Befestigungseinrichtung 36 sich in Richtung auf das Kopfende des Oberteils 31 erstreckenden Befestigungsfortsatz 36 a befestigt, und zwar besonders bevorzugt als offener Griff 2 b mit dem offenen Ende zum Fußende des Bräunungsraums zeigend. Diese Lösung hat den Vorteil, dass der Benutzer die Griff-Vorrichtung ergonomisch gut greifen und zum manuellen Schließen oder Öffnen des Oberteils 31 ergreifen kann, wofür die gesamte Strecke von der höchsten Stelle des Bräunungsraums bis zur Körperoberfläche zur Verfügung steht. Im anderen, ebenfalls bevorzugten Fall ist die Handhabungs-Vorrichtung 1 an einem sich in Richtung auf das Kopfende des Oberteils 31 erstreckenden Befestigungsfortsatz 36 a befestigt, der nicht an der höchsten Stelle der bogenförmigen Befestigungseinrichtung 36 liegt, sondern seitlich davon, und zwar auf der Seite der höchsten Stelle, die der Längsseite des Oberteils 31 gegenüberliegt, an der sich die Gelenke oder Scharniere 23 finden. Diese Lösung hat den Vorteil, dass die Hebelwirkung - relativ zu den Gelenken oder Scharnieren 23 - für den Vorgang des Schließens oder Öffnens des Oberteils aufgrund des größeren Abstandes zum Schwenkpunkt (Gelenke oder Scharniere 23) besser wird und der Vorgang des manuellen Schließens oder Öffnens dabei mit noch weniger Kraftaufwand durchgeführt werden kann.

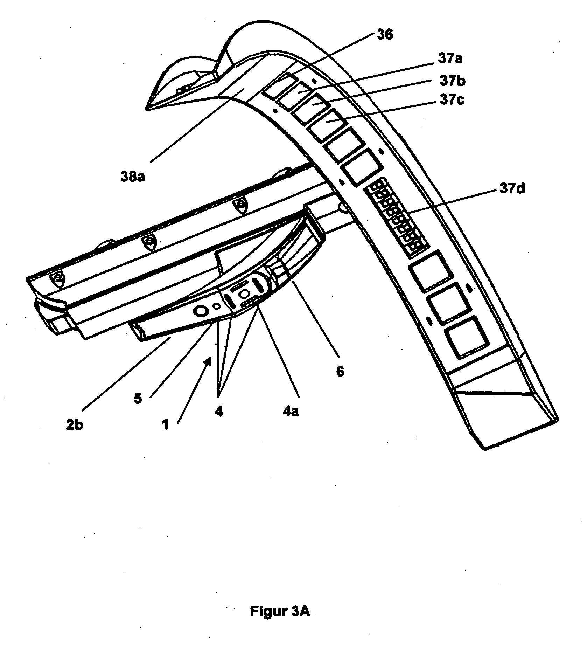

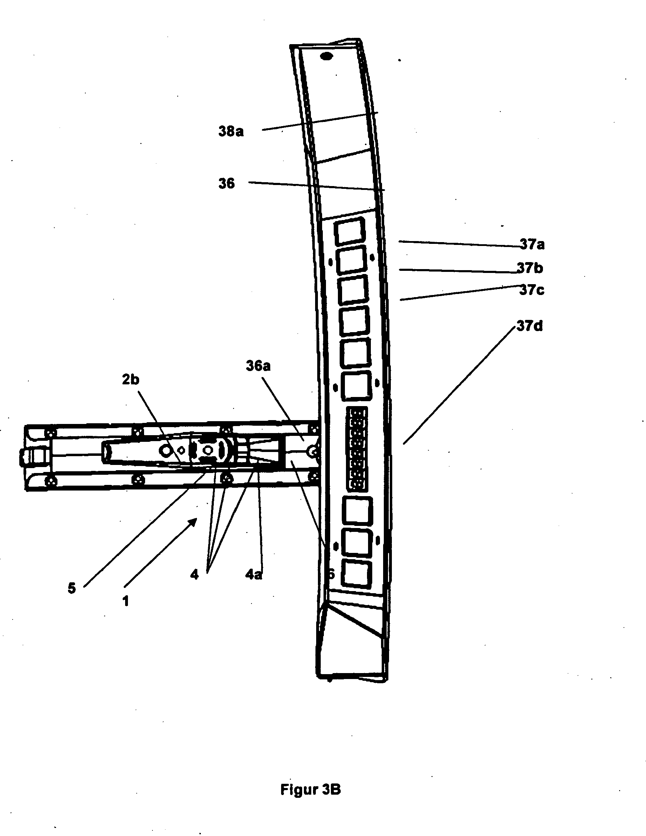

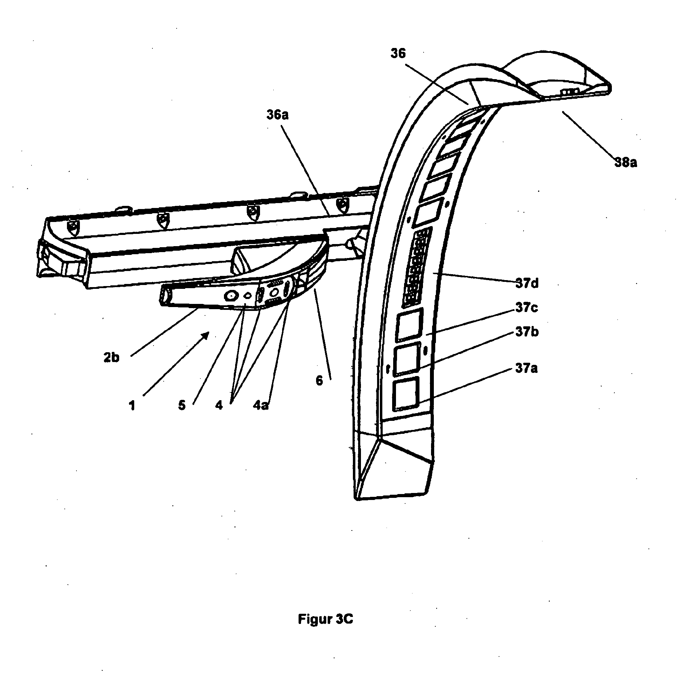

Es ist erfindungsgemäß aufgrund des umfangreichen Zusatznutzens besonders bevorzugt, dass das Bräunungsgerät mit der erfindungsgemäßen Handhabungs-Vorrichtung 1 im Bereich der Griff-Vorrichtung Einrichtungen 4 zum Anwählen und Steuern aller Funktionen im Bräunungsgerät aufweist. Wie sich unter Bezugnahme auf Figuren 3 A bis 3 C ergibt, in denen als bevorzugtes, jedoch nicht beschränkendes Beispiel eine Vorrichtung in Form eines offenen Griffs 2 b gezeigt ist, können in dieser Ausführungsform der Erfindung gleichzeitig mit dem manuellen Schließen des Oberteils 31 des Bräunungsgeräts, jedoch auch zu jedem späteren Zeitpunkt, d. h. auch während des Betriebs des Bräunungsgeräts, alle Funktionen des Bräunungsgeräts sozusagen mit einer Hand angewählt und gesteuert werden. Dies ermöglicht im Gegensatz zu den bisher bekannten Bräunungsgeräten eine schnellere, präzisere und auch für den Benutzer bequemere Anwahl und Steuerung der Geräte-Funktionen. Während nämlich der Benutzer durch Ergreifen der Griff-Vorrichtung mit der Hand in die Lage versetzt wird, das Oberteil 31 des Bräunungsgeräts zu sich hin zu ziehen und dadurch in die Position zu bringen, die einen Start des Bräunungsvorgangs ermöglicht, kann er bereits mit dem Daumen über die Einrichtung 4 die verschiedenen Funktionen des Bräunungsgeräts anwählen, in Fällen der Möglichkeit einer Steuerung die entsprechende Steuerungsfunktion aufrufen und durch einfache Schritte bestätigen bzw. starten.

Dazu sieht die Erfindung in einer noch mehr bevorzugten Ausführungsform ein Bräunungsgerät vor, bei dem die Handhabungs-Vorrichtung 1 als Einrichtungen 4 zum Anwählen und Steuern aller Funktionen im Bräunungsgerät eine Mehrfunktionen-Navigationstaste 4 a mit einer elektronischen Steuerung aufweist, die es ermöglicht, unterschiedliche Programme, die alle Funktionen des Bräunungsgeräts anwählen und steuern, aufzurufen und in den Programmen über Navigier-Schritte einzelne, mehrere oder alle Funktionen zu aktivieren, zu ändern oder zu desaktivieren. Besonders bevorzugt ist - wie aus den Abbildungen 3 A bis 3 C ersichtlich - die Mehrfunktionen-Navigationstaste 4 a eine mit fünf Funktionen versehene Rundknopf-Taste, wie sie auch aus anderen technischen Bereichen (beispielsweise von tragbaren Telefonen oder Spiele-Konsolen oder Multimediagerät-Fernsteuerungen) bekannt ist. Sie weist einen Außenring mit vier Funktionen (bei Vergleich mit einem Kompaß:) bei den vier Himmelsrichtungen Nord, Ost, Süd und West und eine fünfte Funktion durch einen im Zentrum des Außenrings angeordneten "Bestätigungsknopf" auf und ist mit einer elektronischen Steuerung derart verbunden, dass die vier Funktionen des Außenrings ein Navigieren über alle Funktionen des Bräunungsgeräts erlauben und die zentrale fünfte Funktion der Mehrfunktionen-Navigationstaste 4 a die Aktivierung, das Anhalten oder die Desaktivierung der jeweiligen Funktion des Bräunungsgeräts erlaubt. Nicht beschränkende Beispiele verschiedener Funktionen des Bräunungsgeräts, die auf diese Weise durch Navigation anwählbar sind, sind "Gerät (Strahlerröhren) ein/aus", "Zeitschaltung Gerät ein/aus", "Gesichtsbräunerleistung (Stufenschaltung) ein/aus", "Duftspray ja/nein", "Körperlüftung stärker/schwächer", "Klima im Bräuner an/aus/temperaturgenaue Einstellung", und andere.

Eine weitere bevorzugte Ausführungsform der Erfindung betrifft ein Bräunungsgerät, bei dem die erfindungsgemäße Handhabungs-vorrichtung 1 zusätzlich eine Einrichtung zum Stoppen aller Funktionen des Bräunungsgeräts aufweist. In einer noch weiter bevorzugten Ausführungsform der Erfindung ist diese Einrichtung an der Handhabungs-Vorrichtung 1 von den anderen Funktionen getrennt und besonders prominent, so dass sie in jedem Fall intuitiv erkannt und in besonderen Fällen betätigt werden kann. Ein solcher "Not-Aus-Knopf" oder "Not-Aus-Schalter" ist insbesondere dann von Vorteil, wenn aufgrund von Fehlfunktionen oder Problemen mit der Stromversorgung ein Ausschalten des Bräunungsgeräts aus Sicherheitsgründen erfolgen muß, um Schäden an Leib und Leben des Benutzers zu vermeiden.

Weitere bevorzugte Ausführungsformen der Erfindung betreffen ein Bräunungsgerät, dessen erfindungsgemäße Handhabungs-Vorrichtung 1 zusätzlich eine oder mehrere weitere Funktion(en) des Bräunungsgeräts integriert aufweist. Dies können, wie dem Fachmann in diesem technischen Bereich bekannt ist und daher keiner weiteren Erklärung bedarf, sowohl elektrische/elektronische als auch mechanische Funktionen sein.

Besonders bevorzugt ist beispielsweise eine Ausführungsform des erfindungsgemäßen Bräunungsgeräts mit einer zusätzlichen mechanischen Funktion: Die Handhabungs-Vorrichtung 1 weist zusätzlich wenigstens eine integrierte Düse zum Ausstoßen eines Material-Spays in den Bräunungsraum 32 auf. Generell dienen Spraydüsen in Bräunungsgeräten dazu, in den Bräunungsraum 32 ein - vornehmlich der Kühlung des Körpers des Benutzers dienendes, aber gegebenenfalls auch einen angenehmen, den typischen Ozonduft mindernden Duft verbreitendes - Spray einer meist wässrigen Flüssigkeit auszutragen. Dies kann über eine in den Bräunungsraum gerichtete Sprühdüse oder über mehrere derartige Düsen geschehen. Bräunungsgeräte mit mehreren Düsen sind heute üblich, wobei meist mindestens eine Sprühdüse auf den Körper des Benutzers gerichtet ist und mindestens eine Sprühdüse auf das Gesicht des Benutzers gerichtet ist. In einer besonders bevorzugten Ausführungsform der Erfindung ist die auf das Gesicht des Benutzers gerichtete Sprühdüse in die Handhabungs-Vorrichtung 1 integriert und sprüht ein Spray aus einer besonders bevorzugt wässrigen Flüssigkeit in den Kopf-Bereich des Bräunungsraums 32 oder direkt in Richtung auf das Gesicht des Benutzers. Denkbar ist jedoch auch, dass das über die Sprühdüse ausgebrachte Spray andere Funktionen haben kann, beispielsweise die Haut des Benutzers pflegende Funktionen.

In einer weiteren, mit Vorteil einsetzbaren und daher bevorzugten Ausführungsform weist die Handhabungs-Vorrichtung 1 ein weiteres elektrisches bzw. elektronisches Merkmal auf: In dem Fall, in dem das Bräunungsgerät über eine integrierte Multimedia-Anlage (also eine Anlage zum Senden und/oder Empfangen und/oder Wiedergeben von Bild- und/oder Tonsignalen) verfügt, weist die Handhabungs-Vorrichtung 1 zusätzlich wenigstens eine Einrichtung zum Zuschalten der integrierten Multimedia-Anlage auf. Dies ist deswegen besonders bevorzugt, weil das Zuschalten der Multimedia-Anlage gleichzeitig mit dem Schließen des Oberteils 31 des Bräunungsgeräts durch den Benutzer im Liegen erfolgen kann, jedoch auch jederzeit nach dem Schließen des Oberteils des Bräunungsgeräts während des Bräunungsvorgangs, ohne dass sich der Benutzer dazu in eine unbequeme Position begeben muß. In einer weiter bevorzugten Ausführungsform der Erfindung ist die oben bereits erwähnte Mehrfunktionen-Navigationstaste 4 a so geschaltet, dass sie nach Anwählen und Steuern der Funktionen des Bräunungsgeräts und Betätigen der Umschalt-Taste für die Multimedia-Anlage ein Anwählen und Steuern bestimmter Funktionen der Multimedia-Anlage durch Navigation erlaubt. Beispiele hierfür sind (ohne die Erfindung hierauf zu beschränken) die Wahl zwischen verschiedenen Medien (Fernsehen, Zugriff auf Datenträger wie beispielsweise CD-Rom oder Festplatte, Radiosender, Internetzugang einschließlich Herunterladen von Bild- und/oder Tondateien, usw.; sowie innerhalb der verschiedenen Medien Wahl bestimmter Frequenzen (Sender), Datenträger, Homepages usw.; sowie innerhalb der verschiedenen Abteilungen Wahl einzelner Filme, Musikstücke, Homepage-Inhalte usw.).

In einer weiteren vorteilhaften und damit besonders bevorzugten Ausführungsform der Erfindung weist das Bräunungsgerät weitere Einrichtungen auf, mit denen der Benutzer beim Bedienen der Mehrfunktionen-Navigationstaste 4 a an der Handhabungs-Vorrichtung 1 das Ansteuern, die Wahl der Funktionen und die Details der Navigation kontrollieren kann. Derartige Einrichtungen kennt der Fachmann in unterschiedlicher Art, und alle derartigen bekannten Einrichtungen können im Rahmen der vorliegenden Erfindung gewählt werden.

Beispielsweise kann eine derartige Kontroll-Einrichtung eine akustische Einrichtung sein, die dem Benutzer mit akustischen Signalen, besonders bevorzugt sogar mit Sprachsignalen, das Ergebnis seiner Anwahl, Steuerung bzw. Navigation mitteilt und/oder ihn sogar auf Fehler aufmerksam macht oder ihn/sie warnt (letzteres beispielsweise in Fällen, in denen eine bestimmte Wahl keinen Sinn macht oder die Wahl des Benutzers im Rahmen des gegebenen Programms (beispielsweise bei Wahl einer bestimmten Bräunungszeit bei vorgegebener Intensität der verabreichten UV-Strahlung) nicht zulässig ist, beispielsweise deswegen, weil die Bräunungszeit bei der gegebenen Intensität für seinen/ihren Hauttyp nicht zuträglich ist). Dies ist auch deswegen besonders vorteilhaft, weil der Benutzer während des Bräunungsvorgangs gehalten ist, zum Schutz der Augen gegen die intensive sichtbare Strahlung und die UV-Strahlung einen Augenschutz zu tragen, der das optische Erkennen von Informationen zumindest einschränkt.

In einer weiteren besonders bevorzugten Ausführungsform der Erfindung kann die Kontroll-Einrichtung eine Einrichtung sein, die dem Benutzer mit optischen Signalen das Ergebnis seiner Anwahl, Steuerung bzw. Navigation mitteilt. Auch in diesem Fall können optische Signale auf Fehler aufmerksam machen oder warnen. Im Rahmen des erfindungsgemäßen Bräunungsgeräts können die Ergebnisse der Navigation mit der Handhabungs-Vorrichtung 1 beispielsweise im Bereich des Sichtfeldes des Benutzers sichtbar gemacht werden, wenn zusätzlich die über dem Benutzer im Oberteil 31 des Bräunungsgeräts angeordnete bogenförmige Befestigungseinrichtung 36, die sich weiter bevorzugt im Bereich der Schutzfläche 34 des Oberteils 31 befindet und sogar noch mehr bevorzugt direkt oberhalb der Schutzfläche 34 des Oberteils 31 des Bräunungsgeräts befindet, mit einer oder mehreren Anzeige(n) 37 a, 37 b, 37 c ... versehen ist, die einer Erkennung der mit der Mehrfunktionen-Navigationstaste 4 a angesprochenen und zu wählenden Funktion oder sogar mehrerer Funktionen dienen. Es ist ganz besonders bevorzugt, dass mehrere, beispielsweise (ohne Beschränkung) vier oder sechs oder acht derartige Anzeigen vorhanden sind. Dies kann aufgrund der vom Fachmann durchzuführenden Wahl der elektronischen Schaltung beliebig gewählt werden und kann einzelnen Funktionen oder einer Gruppe von Funktionen zugeordnet werden. Diese können (aus Sicht des liegenden Benutzers) übereinander oder nebeneinander angeordnet sein und sind vorzugsweise über die bogenförmige Befestigungseinrichtung verteilt (aus Sicht des Benutzers) nebeneinander angeordnet.

Es entspricht besonders bevorzugten Ausführungsformen der Erfindung, dass die eine oder die mehreren Anzeige(n) 37 a, 37 b, 37 c, ... ein Piktogramm oder mehrere Piktogramme ist/sind, also Anzeigen, die ohne Worte/Erläuterungen leicht erkennen lassen, welche Funktion gerade angewählt ist oder gesteuert wird. Figuren 3 A bis 3 C zeigen eine solche Anordnung. Dazu sind die Anzeigen beispielsweise mit leicht verständlichen Symbolen versehen, die das Verständnis des Navigationsvorgangs erleichtern und beschleunigen. Bevorzugterweise ist das/sind die Anzeige(n)/Piktogramm(e) hinterleuchtet, d. h. hinter der Oberfläche mit einem oder mehreren aktivierbaren und desaktivierbaren, vorzugsweise mit einem oder mehreren elektronisch steuerbaren, Leuchtmittel(n) versehen, das/die es dem Benutzer erlaubt/erlauben, selbst bei Tragen eines Augenschutzes und/oder hellem Licht der UV-Strahlung abgebenden Lichtquellen zu erkennen, in welchem Teil des mit der Mehrfunktionen-Navigationstaste 4 a steuerbaren Menus er sich befindet. Es ist wegen der besondes guten Erkennbarkeit von großem Vorteil, wenn das/die Anzeige(n)/Piktogramm(e) von einer oder mehreren, vorzugsweise Licht unterschiedlicher Farben/Wellenlängen emittierenden LED's hinterleuchtet sind.

Wegen der guten Erkennbarkeit auch mit Augenschutz und/oder bei dem hellen, von den UV-Strahlungsquellen emittierten Licht ist es dabei besonders bevorzugt, dass die LED-Hinterleuchtung des/der Piktogramm(e) so gewählt ist, dass die Erkennbarkeit des/der aktivierten Piktogramme(s) sowohl bei noch nicht in Betrieb befindlichem als auch bei in Betrieb befindlichem Bräunungsgerät gewährleistet ist. Ohne die Erfindung hierauf zu beschränken, kann dies dadurch erreicht werden, dass man beispielsweise mehrere sogenannte Flux-LED's vorsieht, von denen beispielsweise (ohne die Erfindung durch dieses Beispiel zu beschränken) einige rot sind, einige weiß und einige blau sein können. Um selbst bei laufendem Betrieb des Bräunungsgeräts die Erkennbarkeit der mit der Multifunktionen-Navigationstaste 4 a gewählten Funktionen zu gewährleisten, werden mittels der genannten LED's bestimmte Farben des Piktogramms, das einem bestimmten Programm-Punkt oder -Unterpunkt entspricht, eingeschaltet und andere ausgeschaltet.

Es entspricht einer weiteren bevorzugten Ausführungsform der Erfindung, bei dem Bräunungsgerät zusätzlich ein mehrstelliges Display 37 d zur lesbaren Information des Benutzers über die aktuell mittels der Mehrfunktions-Navigationstaste 4 a gewählten Parameter vorzusehen. Dies ermöglicht es dem Benutzer in vorteilhafter Weise, zusätzlich zu den Piktogrammen konkrete Informationen über den aktuellen Status und die gewählten Parameter zu erhalten, sozusagen "im Klartext". Ein derartiges Display kenn jedes beliebige, dem Fachmann bekannte Display sein, wird jedoch in bevorzugten Ausführungsformen ein Display sein, dessen Erkennbarkeit unter den in einem im Betrieb befindlichen Bräunungsgerät herrschenden Bedingungen gewährleistet ist, insbesondere was die gewählten Farben und den Kontrast zum Licht der UV-Strahlungsquellen angeht. Zur Verbesserung der Erkennbarkeit ist das Display 37 d vorzugsweise zentral im Blickfeld des Benutzers angeordnet, beispielsweise in der Mitte der in dem Bräunungsraum 32 befindlichen bogenförmigen Befestigungseinrichtung 36, besonders bevorzugt in der Mitte der oben näher beschriebenen Piktogramme.

Vorzugsweise ist/sind die eine oder mehreren Anzeige(n) 37 a, 37 b, 37 c ... oberhalb der Schutzfläche 34 angeordnet, die sich (bei Betrieb des Bräunungsgeräts) oberhalb des Benutzers im Oberteil 31 des Bräunungsgeräts befindet. Dies schützt nicht nur die Anzeige(n) und ihre Elektronik wie auch gegebenenfalls die Leuchtmittel, die sie hinterleuchten, vor mechanischer Beschädigung, sondern erleichtert auch das Reinigen und/oder Desinfizieren der Schutzfläche 34 nach dem Bräunungsvorgang, da eine durchgehende Fläche bearbeitet werden kann, ohne befürchten zu müssen, dass sich Desinfektionsmittel und/oder Reinigungslösung in die Nähe der Elektrik/Elektronik vorarbeitet.

Weiter bevorzugt ist erfindungsgemäß ein Bräunungsgerät mit einem oder mehreren, vorzugsweise mit allen oben näher beschriebenen Merkmalen, bei dem zusätzlich die im Bereich der Schutzfläche 34 des Oberteils 31, im Bereich der Sicht des Benutzers, angeordnete bogenförmige Befestigungseinrichtung 36 mit einer oder mehreren Anzeige(n) 38 a, 38 b, ... für nicht funktionsgebundene Zwecke versehen ist. Eine solche Anordnung ist nicht nur aus technischer Sicht vorteilhaft, da sich die Befestigungseinrichtung bogenförmig im Bereich der Sicht des Benutzers über den ganzen Bogen der Oberteils 31 des Bräunungsgeräts erstrecken kann, sondern auch aus ästhetischer Sicht, da die technischen Merkmale des Befestigungselements hinter einem (vornehmlich optischen Zwecken dienenden) Anzeige-Element mit einer oder mehreren Anzeige(n) verborgen werden kann. Unter dem Begriff "nicht funktionelle Zwecke" wird in der vorliegenden Beschreibung und in den Patentansprüchen ein Zweck

(oder werden mehrere Zwecke) verstanden, der/die sich nicht auf technische Funktionen des Bräunungsgeräts und/oder auf den technischen Ablauf des Bräunungsvorgangs bezieht/beziehen (wie beispielsweise das Einschalten bestimmter Funktionen des Geräts, die Bräunungszeit, ein bestimmtes Bräunungsprogramm, Zusatzfunktionen wie Lüfter (und dessen Intensität), Sprüh (und dessen Intensität) und/oder Multimedia-Features und/oder die Uhrzeit). Vielmehr können für die nicht funktionsgebundene Anzeige(n) vorzugsweise Angaben wie die Geräte-Marke, der Geräte-Typ, der Reinigungsstatus des Geräts ("für Sie frisch gereinigt") oder ähnliches vorgesehen werden. Denkbar ist in einer weiteren, zusätzlich verwirklichbaren Ausführungsform auch, dass die Anzeige(n) für Werbezwecke verwendet wird/werden.

In vorteilhaften Ausführungsformen ist/sind in dem erfindungsgemäßen Bräunungsgerät die eine oder mehreren Anzeige(n) 38 a, 38 b ... für nicht funktionsgebundene Zwecke an der bogenförmigen Befestigungseinrichtung 36 so angeordnet, dass die optisch den Bogen der Funktionen-Anzeigen 37 a, 37 b, 37 c, .... zur Seite des Oberteils 31 des Bräunungsgeräts fortsetzt/fortsetzen, und sind weiter bevorzugt so angeordnet, dass sie für den Benutzer bei geöffnetem Oberteil 31 des Bräunungsgeräts vor dem Platznehmen auf dem Bräunungsgerät von außen erkennbar ist/sind. Dies hat den Vorteil, dass beispielsweise der Reinigungsstatus des Geräts oder der Typ des Geräts für den Benutzer vor dem Platznehmen verifiziert werden kann, beispielsweise wenn es um ein auf das Gerät abgestimmtes Bräunungsprogramm geht oder wenn die Frage relevant ist, ob das Gerät für den Bräunungsvorgang bereits vorbereitet (beispielsweise gereinigt und desinfiziert) wurde.

Die Erfindung betrifft auch eine Handhabungs-Vorrichtung 1, insbesondere eine Griff-Vorrichtung zum Verschließen oder Öffnen des Oberteils 31 eines wie vorstehend beschriebenen Bräunungsgeräts, die am Oberteil 31 angebracht ist und in einer bevorzugten Ausführungsform im Bereich der Schutzfläche 34 im Bereich der Sicht des Benutzers an einem sich von einer bogenförmigen Befestigungsvorrichtung 36 in Richtung auf das Kopfende des Bräunungsgeräts erstreckenden Befestigungsfortsatz 36 a konkavseitig angebracht ist und dazu dient, dass der Benutzer des Bräunungsgeräts das Oberteil nach dem Platznehmen auf dem Bräunungsgerät im Liegen schließen oder öffnen und gegebenenfalls gleichzeitig mit dem Schließ- oder Öffnungsvorgang oder danach, einschließlich während des Ablaufs des Bräunungsvorgangs, Funktionen des Bräunungsgeräts über die Handhabungs-Vorrichtung 1 ansteuern, auswählen, aktivieren oder desaktivieren und so in den Programm-Funktionen navigieren kann.

Die einzelnen Merkmale der erfindungsgemäßen Handhabungs-Vorrichtung bzw. Handhabungs-Vorrichtung 1 wurden oben im Detail im Zusammenhang mit dem erfindungsgemäßen Bräunungsgerät beschrieben. Zur Vermeidung von Wiederholungen wird für die beschriebenen Merkmale der Handhabungs-Vorrichtung bzw. Handhabungs-Vorrichtung 1 auf die obige Beschreibung verwiesen.

Die Erfindung wird durch die in der Beschreibung der bevorzugten Ausführungsformen genannten bevorzugten Merkmale nicht beschränkt. Vielmehr dient die Beschreibung lediglich der Erläuterung der Erfindung.

## Patentansprüche

1. Bräunungsgerät (10), umfassend
(a) ein standfestes Unterteil (21) mit Kopfende und Fußende und zwei Längsseiten (22), enthaltend elektrische Aggregate, Schalt-Einrichtungen und Verbindungs-Einrichtungen; und
(b) ein entweder an einer der Längsseiten (22) des Unterteils (21) über Scharniere oder Gelenke (23) angebrachtes, über die Scharniere oder Gelenke (23) von dem Unterteil (21) weg und auf das Unterteil (21) hin zur Bildung eines Bräunungsraumes (32) klappbares oder schwenkbares oder an einer separaten Aufhängung von dem Unterteil (21) weg hebbares und auf das Unterteil (21) hin zur Bildung eines Bräunungsraumes (32) senkbares Oberteil (31) mit Kopfende und Fußende und zwei Längsseiten; wobei
(c) das Unterteil (21) eine zumindest für bräunende UV-Strahlung durchlässige Liegefläche (24) für den Benutzer und unterhalb der Liegefläche (24) mindestens einen Satz in Längsrichtung des Bräunungsgeräts (10) angeordneter Strahler (25) und gegebenenfalls noch weitere Strahler (25) zur Abgabe von Strahlung mit zumindest einem Anteil an bräunender UV-Strahlung in Richtung auf das Oberteil (31) und/oder zumindest einen Teil des Körpers des Benutzers gerichtet aufweist und das Unterteil (21) die für den Betrieb des Bräunungsgeräts (10) erforderlichen elektrischen Aggregate, Schalt-Einrichtungen und/oder Verbindungs-Einrichtungen aufweist; und
(d) das Oberteil (31) eine zumindest für bräunende UV-Strahlung durchlässige Schutzfläche (34) für den Benutzer und oberhalb der Schutzfläche (34) mindestens einen Satz in Längsrichtung des Bräunungsgeräts (10) angeordneter Strahler (35) und gegebenenfalls noch weitere Strahler (35) zur Abgabe von Strahlung mit zumindest einem Anteil an bräunender UV-Strahlung in Richtung auf das Unterteil (21) und/oder zumindest einen Teil des Körpers des Benutzers gerichtet aufweist;
und umfassend
(e) an seinem Oberteil (31) eine durch den Benutzer manuell bedienbare Handhabungs-Vorrichtung (1) zum manuellen Handhaben einschließlich Schließen und Öffnen des Bräunungsgeräte-Oberteils, wobei die Handhabungs-Vorrichtung (1) Einrichtungen (4) zum Anwählen und Steuern zumindest der Funktionen Start des Bräunungsgeräts (10) und Verlauf des Bräunungsvorgangs des Bräunungsgeräts (10) aufweist.

2. Bräunungsgerät (10) nach Anspruch 1, umfassend im Bereich der Schutzfläche (34) des Oberteils (31) eine bogenförmige Befestigungseinrichtung (36) mit einem sich in Richtung auf das Kopfende erstreckenden Befestigungsfortsatz (36 a), an dem die manuell bedienbare Handhabungs-Vorrichtung (1) konkavseitig angebracht ist.

3. Bräunungsgerät (10) nach Anspruch 1 oder Anspruch 2, worin die Handhabungs-Vorrichtung (1) in Form eines U-förmigen Griffs (2 a) oder eines offenen Griffs (2 b) oder eines stabartigen Griffs (2 c) oder eines knaufartigen Griffs (2 d) vorliegt.

4. Bräunungsgerät (10) nach Anspruch 3, worin die Handhabungs-Vorrichtung (1) in Form eines offenen, in Längsrichtung des Bräunungsraums (32) mit dem offenen Ende zum Fußende des Bräunungsraums (32) angeordneten Griffs vorliegt.

5. Bräunungsgerät (10) nach Anspruch 2 oder Anspruch 3, worin die Handhabungs-Vorrichtung (1) an dem an der höchsten Stelle der bogenförmigen Befestigungseinrichtung (36) sich in Richtung auf das Kopfende des Oberteils (31) erstreckenden Befestigungsfortsatz (36 a) befestigt ist, oder worin die Handhabungs-Vorrichtung (1) an einem seitlich der höchsten Stelle der bogenförmigen Befestigungseinrichtung (36) auf der Seite, die der Seite der Gelenke oder Scharniere (23) gegenüber liegt, angebrachten, sich in Richtung auf das Kopfende des Oberteils (31) erstreckenden Befestigungsfortsatz (36 a) befestigt ist.

6. Bräunungsgerät (10) nach einem der Ansprüche 1 bis 5, worin die Handhabungs-Vorrichtung (1) als Einrichtungen (4) zum Anwählen und Steuern von Funktionen in einem Bräunungsgerät (10) eine Mehrfunktionen-Navigationstaste (4 a) mit einer elektronischen Steuerung aufweist, die es ermöglicht, unterschiedliche Programme, die alle Funktionen des Bräunungsgeräts (10) anwählen und steuern, aufzurufen und in den Programmen über Navigier-Schritte einzelne, mehrere oder alle Funktionen zu aktivieren, zu ändern oder zu desaktivieren.

7. Bräunungsgerät (10) nach einem der Ansprüche 1 bis 6, worin die Handhabungs-Vorrichtung (1) weiter eine Einrichtung zum Stoppen aller Funktionen des Bräunungsgeräts aufweist, und/oder worin die Handhabungs-Vorrichtung (1) eine oder mehrere Funktion(en) des Bräunungsgeräts integriert aufweist.

8. Bräunungsgerät (10) nach Anspruch 7, worin die Handhabungs-Vorrichtung (1) zusätzlich wenigstens eine integrierte Düse zum Ausstoßen eines Material-Spays in den Bräunungsraum (32) aufweist.

9. Bräunungsgerät (10) nach einem der Ansprüche 6 bis 8, worin die Handhabungs-Vorrichtung (1) zusätzlich wenigstens eine Einrichtung zum Zuschalten einer in das Bräunungsgerät (10) integrierten Multimedia-Anlage aufweist.

10. Bräunungsgerät (10) nach Anspruch 9, worin die Handhabungs-Vorrichtung (1) dafür geeignet ist, dass nach Betätigen der Einrichtung zum Zuschalten einer in das Bräunungsgerät (10) integrierten Multimedia-Anlage die Mehrfunktionen-Navigationstaste (4a) der Handhabungs-Vorrichtung (1) das Navigieren in der Multimedia-Anlage erlaubt.

11. Bräunungsgerät nach einem der Ansprüche 2 bis 10, worin zusätzlich die im Bereich der Schutzfläche (34) des Oberteils (31) angeordnete bogenförmige Befestigungseinrichtung (36) mit einer Anzeige oder mehreren Anzeigen (37 a, 37 b, 37 c) zur Erkennbarkeit der mit der Mehrfunktionen-Navigationstaste (4 a) angesprochenen und zu wählenden Funktion oder Funktionen versehen ist.

12. Bräunungsgerät (10) nach Anspruch 11, worin die eine Anzeige oder mehreren Anzeigen ein Piktogramm oder mehrere Piktogramme ist/sind, das/die mit LED's hinterleuchtete Piktogramme ist/sind, worin die LED-Hinterleuchtung des Piktogramms/ der Piktogramme so gewählt ist, dass die Erkennbarkeit des aktivierten Piktogramms /der aktivierten Piktogramme sowohl bei noch nicht in Betrieb befindlichem als auch bei in Betrieb befindlichem Bräunungsgerät (10) gewährleistet ist.

13. Bräunungsgerät (10) nach Anspruch 6, umfassend zusätzlich ein vorzugsweise mehrstelliges Display (37 d) zur lesbaren Information des Benutzers über die aktuell mittels der Mehrfunktions-Navigationstaste (4 a) gewählten Parameter.

14. Bräunungsgerät (10) nach einem der Ansprüche 11 bis 13, worin die eine Anzeige oder die mehreren Anzeigen oberhalb der Schutzfläche (34) angeordnet ist/sind.

15. Bräunungsgerät (10) nach einem der Ansprüche 2 bis 14, worin zusätzlich die im Bereich der Schutzfläche (34) des Oberteils (31) angeordnete bogenförmige Befestigungseinrichtung (36) mit einer Anzeige oder mehreren Anzeigen (38 a, 38 b) für nicht funktionsgebundene Zwecke versehen ist.

16. Oberteil für ein Bräunungsgerät, welches entweder an einer der Längsseiten (22) des Unterteils (21) eines Bräunungsgeräts (10) über Scharniere oder Gelenke (23) anbringbar, über die Scharniere oder Gelenke (23) von dem Unterteil (21) weg und auf das Unterteil (21) hin zur Bildung eines Bräunungsraumes (32) klappbar oder schwenkbar oder an einer separaten Aufhängung von dem Unterteil (21) eines Bräunungsgeräts (10) weg hebbar und auf das Unterteil (21) hin zur Bildung eines Bräutiungsraumes (32) senkbar ist, wobei das Oberteil (31) ein Kopfende, ein Fußende, zwei Längsseiten und eine Handhabungs-Vorrichtung (1) zum manuellen Handhaben einschließlich Schließen und Öffnen des Oberteils (31) aufweist, wobei die Handhabungs-Vorrichtung (1) am Oberteil (31) des Bräunungsgeräts (10) an einer für den Benutzer erreichbaren Stelle anbringbar ist und Einrichtungen (4) zum Anwählen und Steuern zumindest der Funktionen Start des Bräunungsgeräts (10) und Verlauf des Bräunungsvorgangs des Bräunungsgeräts (10) aufweist.

17. Oberteil (31) für ein Bräunungsgerät (10) mit Handhabungs-Vorrichtung (1) nach Anspruch 16, anbringbar am Oberteil (31) des Bräunungsgeräts (10) im Bereich der Schutzfläche (34) an einer bogenförmigen Befestigungseinrichtung (36) mit einem sich in Richtung auf das Kopfende erstreckenden Befestigungsfortsatz (36 a).

18. Oberteil (31) für ein Bräunungsgerät (10) mit Handhabungs-Vorrichtung (1) nach Anspruch 16 oder Anspruch 17 mit der Handhabungs-Vorrichtung (1) in Form eines U-förmigen Griffs (2 a) oder eines offenen Griffs (2 b) oder eines stabartigen Griffs (2c) oder eines knaufartigen Griffs (2d) oder einer in der Schutzfläche 34 eingelassenen Griffmulde (2e).

19. Oberteil (31) für ein Bräunungsgerät (10) mit Handhabungs-Vorrichtung (1) nach Anspruch 18 mit der Handhabungs-Vorrichtung (1) in Form eines offenen, in Längsrichtung des Bräunungsraums (32) mit dem offenen Ende zum Fußende des Bräunungsraums (32) angeordneten Griffs (2 b).

20. Oberteil (31) für ein Bräunungsgerät (10) mit Handhabungs-Vorrichtung (1) nach Anspruch 16, 17 oder 18, wobei die Handhabungs-Vorrichtung (1) an dem an der höchsten Stelle der bogenförmigen Befestigungseinrichtung (36) sich in Richtung auf das Kopfende des Oberteils (31) erstreckenden Befestigungsfortsatz (36 a) befestigt ist oder an dem seitlich der höchsten Stelle der bogenförmigen Befestigungseinrichtung (36) auf der Seite, die der Seite der Gelenke oder Scharniere (23) gegenüber liegt, angebrachten, sich in Richtung auf das Kopfende des Oberteils (31) erstreckenden Befestigungsfortsatz (36 a) befestigt ist.

21. Oberteil (31) für ein Bräunungsgerät (10) mit Handhabungs-Vorrichtung (1) nach einem der Ansprüche 16 bis 20, wobei die Handhabungs-Vorrichtung (1) als Einrichtungen (4) zum Anwählen und Steuern von Funktionen in einem Bräunungsgerät eine Mehrfunktionen-Navigationstaste (4a) mit einer elektronischen Steuerung aufweist, die es ermöglicht, unterschiedliche Programme, die alle Funktionen des Bräunungsgeräts anwählen und steuern, aufzurufen und in den Programmen über Navigier-Schritte einzelne, mehrere oder alle Funktionen zu aktivieren, zu ändern oder zu desaktivieren.

22. Oberteil (31) für ein Bräunungsgerät (10) mit Handhabungs-Vorrichtung (1) nach einem der Ansprüche 16 bis 21, wobei die Handhabungs-Vorrichtung (1) weiter eine Einrichtung zum Stoppen aller Funktionen des Bräunungsgeräts (10) aufweist.

23. Oberteil (31) für ein Bräunungsgerät (10) mit Handhabungs-Vorrichtung (1) nach einem der Ansprüche 16 bis 22, wobei die Handhabungs-Vorrichtung (1) zusätzlich eine oder mehrere Funktionen des Bräunungsgeräts integriert aufweist.

24. Oberteil (31) für ein Bräunungsgerät (10) mit Handhabungs-Vorrichtung (1) nach Anspruch 23, wobei die Handhabungs-Vorrichtung (1) zusätzlich eine integrierte Düse zum Ausstoßen eines wässrigen Sprays in den Kopf-Bereich des Bräunungsraums (32) aufweist.

25. Oberteil (31) für ein Bräunungsgerät (10) mit Handhabungs-Vorrichtung (1) nach Anspruch 23, wobei die Handhabungs-Vorrichtung (1) zusätzlich wenigstens eine Einrichtung zum Zuschalten einer in das Bräunungsgerät integrierten Multimedia-Anlage aufweist.

26. Oberteil (31) für ein Bräunungsgerät (10) mit Handhabungs-Vorrichtung (1) nach Anspruch 25, worin nach Betätigen der Einrichtung zum Zuschalten einer in das Bräunungsgerät integrierten Multimedia-Anlage die Mehrfunktionen-Navigationstaste (4a) der Griff-Vorrichtung (1) das Navigieren in der Multimedia-Anlage erlaubt.

## Claims

1. A tanning device (10), comprising
(a) a stationary lower part (21) having a head end and a foot end and two longitudinal sides (22), containing electrical units, switching means and connecting means; and
(b) an upper part (31) having a head end and a foot end and two longitudinal sides, either fitted to one of the longitudinal sides (22) of the lower part (21) by means of hinges or joints (23), tiltable or swivellable by means of said hinges or joints (23) away from the lower part (21) and towards the lower part (21) to form a tanning chamber (32) or liftable away from the lower part (21) and lowerable towards the lower part (21) to form a tanning chamber (32) by means of a separate suspension; wherein
(c) the lower part (21) comprises a recumbency surface (24) for the user which is permeable at least to tanning UV radiation and at least one set of lamps (25) arranged beneath the recumbency surface (24) in the longitudinal direction of the tanning device (10) and optionally also further lamps (25) for outputting radiation with at least a proportion of tanning UV radiation towards the upper part (31) and/or at least a part of the user's body, said lower part (21) having the electrical units, switching means and/or connecting means required for the operation of the tanning device (10); and
(d) the upper part (31) comprises a protective surface (34) for the user permeable at least to tanning UV radiation and at least one set of lamps (35) arranged above the protective surface (34) in the longitudinal direction of the tanning device (10) and optionally also further lamps (35) for outputting radiation with at least a proportion of tanning UV radiation towards the lower part (21) and/or at least a part of the user's body;
and comprising
(e) at the upper part (31) an operating device (1) for manual operation, by the user, for manually operating, including manually closing and opening, of the tanning device upper part, whereby the operating device (1) has means (4) for addressing and controlling at least the functions of starting the tanning device (10) and of the course of the tanning operation of the tanning device (10).

2. The tanning device (10) of claim 1, comprising an arcuated fixation means (36) in the area of the protective surface (34) of the upper part (31) and having a fixation extension (36a) extending towards the head end, to the concave side of which said manually operable operating device (1) is applied.

3. The tanning device (10) according to claim 1 or claim 2, wherein the operating device (1) has the shape of a U-shape grip (2a) or of an open grip (2b) or of a rod-like grip (2c) or of a stud-like grip (2d).

4. The tanning device (10) according to claim 3, wherein the operating device (1) has the shape of an open grip arranged in the longitudinal direction of the tanning chamber (32) and has its open end directed towards the foot end of the tanning chamber (32).

5. The tanning device (10) according to claim 2 or claim 3, wherein the operating device (1) is mounted to the fixation extension (36a) provided at the highest level of the arcuated fixation means (36) and extending towards the head end of the upper part (31), or wherein the operating device (1) is mounted to the fixation extension (36a) mounted laterally to the highest level of the arcuated fixation means (36) on the side opposite to the side of the hinges or joints (23) and extending towards the head end of the upper part (31).

6. The tanning device (10) according to any of the claims 1 to 5, wherein the operating device (1) has a multi-function navigation key (4a) with an electronic control as the means (4) for addressing and controlling functions in a tanning device (10), which key allows accessing different programs addressing and controlling all functions of the tanning device (10) and which allows activating, changing or deactivating single, plural or all functions via navigation steps.

7. The tanning device (10) according to any of the claims 1 to 6, wherein said operating device (1) further has means for stopping all functions of the tanning device, and/or wherein said operating device (1) further has one or several function(s) of the tanning device integrated.

8. The tanning device (10) according to claim 7, wherein said operating device (1) further has at least an integrated nozzle for ejecting a material spray into the tanning chamber (32).

9. The tanning device (10) according to any of the claims 6 to 8, wherein said operating device (1) further has at least means for additionally switching on multi-media systems integrated into the tanning device (10).

10. The tanning device (10) according to claim 9, wherein the operating device (1) is suitable for allowing, after activating said means for additionally switching on multi-media systems integrated into the tanning device (10), a navigation in the multi-media system by means of the multi-function navigation key (4a).

11. The tanning device (10) according to any of the claims 2 to 10, wherein further the arcuated fixation means (36) mounted in the area of the protective surface (34) of the upper part (31) is provided with a display or several displays (37a, 37b, 37c) for securing recognizability of the function or functions addressed by and to be selected by the multi-function navigation key (4a).

12. The tanning device (10) according to claim 11, wherein the display or the several displays is/are a pictogram or several pictograms which is/are (a) pictogram(s) back-lighted with LED's, wherein the back-lightening, by LED's, of the pictogram(s) is selected such that the recognizability of the activated pictogram / of the activated pictograms is secured when the tanning device (10) is not yet operated and also when the tanning device (10) is operated.

13. The tanning device (10) according to claim 6, comprising further a display (37d) which preferably is a multi-digit display for informing, by readable information, the user about the parameters currently selected by means of the multi-function navigation key (4a).

14. The tanning device (10) according to any of the claims 11 to 13, wherein one display or several displays are arranged above the protective surface (34).

15. The tanning device (10) according to any of the claims 2 to 14, wherein further the arcuated fixation means (36) mounted in the area of the protective surface (34) of the upper part (31) is provided with a display or several displays (38a, 38b) for purposes not connected to the functions of the device.

16. An upper part for a tanning device which is either fittable to one of the longitudinal sides (22) of the lower part (21) of a tanning device (10) by means of hinges or joints (23), tiltable or swivellable by means of said hinges or joints (23) away from the lower part (21) and towards the lower part (21) to form a tanning chamber (32) or liftable away from the lower part (21) and lowerable towards the lower part (21) of a tanning device (10) to form a tanning chamber (32) by means of a separate suspension, whereby the upper part (31) has a head end, a foot end, two longitudinal sides and an operating device (1) for manually operating, including opening and closing, the tanning device upper part (31), wherein the operating device (1) is mountable to the upper part (31) of the tanning device (10) at a location reachable by the user and has means (4) for addressing and controlling at least of the functions "start" of the tanning device and course of operation of the tanning process of the tanning device (10).

17. The upper part (31) for a tanning device (10) having an operation device (1) according to claim 16, mountable to the upper part (31) of the tanning device (10) in the area of the protective surface (34) at an arcuated fixation means (36) by means of a fixation extension (36a) extending towards the head end.

18. The upper part (31) for a tanning device (10) having an operation device (1) according to claim 16 or claim 17 and having an operation device (1) having the shape of a U-shape grip (2a) or of an open grip (2b) or of a rod-like grip (2c) or of a stud-ike grip (2d) or of a recessed grip embedded into the protective surface (34).

19. The upper part (31) for a tanning device (10) having an operation device (1) according to claim 18 and having an operation device (1) having the shape of an open grip (2b) arranged in the longitudinal direction of the tanning chamber (32) with its open end towards the foot end of the tanning chamber (32).

20. The upper part (31) for a tanning device (10) having an operation device (1) according to claim 16, 17 or 18, wherein the operating device (1) is mounted to the fixation extension (36a) provided at the highest level of the arcuated fixation means (36) and extending towards the head end of the upper part (31), or wherein the operating device (1) is mounted to the fixation extension (36a) mounted laterally to the highest level of the arcuated fixation means (36) on the side which is opposite to the side of the hinges or joints (23) and extending towards the head end of the upper part (31).

21. The upper part (31) for a tanning device (10) having an operation device (1) according to any of the claims 16 to 20, wherein the operating device (1) has a multi-function navigation key (4a) with an electronic control as the means (4) for addressing and controlling functions in a tanning device, which key allows accessing different programs addressing and controlling all functions of the tanning device and which allows activating, changing or deactivating single, plural or all functions via navigation steps.

22. The upper part (31) for a tanning device (10) having an operation device (1) according to any of the claims 16 to 21, wherein the operating device (1) further has means for stopping all functions of the tanning device (10).

23. The upper part (31) for a tanning device (10) having an operation device (1) according to any of the claims 16 to 22, wherein said operating device (1) further has one or several function(s) of the tanning device integrated.

24. The upper part (31) for a tanning device (10) having an operation device (1) according to claim 23, wherein said operating device (1) further has an integrated nozzle for ejecting an aqueous spray into the head area of the tanning chamber (32).

25. The upper part (31) for a tanning device (10) having an operation device (1) according to claim 23, wherein said operating device (1) further has at least a means for additionally switching on multi-media systems integrated into the tanning device.

26. The upper part (31) for a tanning device (10) having an operation device (1) according to claim 25, wherein, after activating said means for additionally switching on multi-media systems integrated into the tanning device, the multi-function navigation key (4a) of the grip device (1) allows a navigation in the multi-media systems.

## Revendications

1. Appareil de bronzage (10), comprenant
(a) une partie inférieure fixe (21) comportant une extrémité pour la tête et une extrémité pour les pieds et deux côtés longitudinaux (22), contenant des agrégats électriques, des dispositifs de commutation et des dispositifs de connexion ; et
(b) une partie supérieure (31) comportant une extrémité pour la tête et une extrémité pour les pieds et deux côtés longitudinaux qui peut soit être montée sur un des côtés longitudinaux (22) de la partie inférieure (21) par le biais de charnières ou d'articulations (23) et peut, en basculant ou en pivotant par le biais des charnières ou articulations (23), être éloignée de la partie inférieure (21) et rabattue sur la partie inférieure (21) pour former un compartiment de bronzage (32), soit être éloignée de la partie inférieure (21) par élévation et abaissée sur la partie inférieure (21), pour former un compartiment de bronzage (32), sur une suspension séparée ;
(c) la partie inférieure (21) étant pourvue d'une surface d'allongement (24) perméable au moins au rayonnement UV bronzant pour l'utilisateur et, sous la surface d'allongement (24), d'au moins un ensemble d'émetteurs (25) disposés dans la direction longitudinale de l'appareil de bronzage (10) et, le cas échéant, encore d'autres émetteurs (25) destinés à émettre un rayonnement avec au moins une partie de rayonnement UV bronzant orientée en direction de la partie supérieure (31) et/ou d'au moins une partie du corps de l'utilisateur, et la partie inférieure (21) étant pourvue des agrégats électriques, des dispositifs de commutation et/ou des dispositifs de connexion nécessaires pour le fonctionnement de l'appareil de bronzage (10);et
(d) la partie supérieure (31) étant pourvue d'une surface de protection (34) perméable au moins au rayonnement UV bronzant pour l'utilisateur et, au-dessus de la surface de protection (34), d'au moins un ensemble d'émetteurs (35) disposés dans la direction longitudinale de l'appareil de bronzage (10) et, le cas échéant, encore d'autres émetteurs (35) destinés à émettre un rayonnement avec au moins une partie de rayonnement UV bronzant orientée en direction de la partie inférieure (21) et/ou d'au moins une partie du corps de l'utilisateur ;
et comprenant
(e) au niveau de sa partie supérieure (31), un dispositif de manipulation (1) pouvant être actionné manuellement par l'utilisateur en vue d'une manipulation manuelle, y compris la fermeture et l'ouverture, de la partie supérieure de l'appareil de bronzage, le dispositif de manipulation (1) comprenant des dispositifs (4) pour sélectionner et commander au moins les fonctions Mise en marche de l'appareil de bronzage (10) et Déroulement du processus de bronzage de l'appareil de bronzage (10).

2. Appareil de bronzage (10) selon la revendication 1, comprenant, dans la zone de la surface de protection (34) de la partie supérieure (31), un dispositif de fixation en arc (36) présentant un prolongement de fixation (36a) s'étendant en direction de l'extrémité pour la tête, sur lequel le dispositif de manipulation (1) actionnable manuellement est monté sur le côté concave.

3. Appareil de bronzage (10) selon la revendication 1 ou la revendication 2, dans lequel le dispositif de manipulation (1) se présente sous la forme d'une poignée en U (2a) ou d'une poignée ouverte (2b) ou d'une poignée similaire à une barre (2c) ou d'une poignée similaire à un bouton de porte (2d).

4. Appareil de bronzage (10) selon la revendication 3, dans lequel le dispositif de manipulation (1) se présente sous la forme d'une poignée ouverte disposée dans la direction longitudinale du compartiment de bronzage (32) avec son extrémité ouverte dirigée vers l'extrémité pour les pieds du compartiment de bronzage (32).

5. Appareil de bronzage (10) selon la revendication 2 ou la revendication 3, dans lequel le dispositif de manipulation (1) est fixé sur le prolongement de fixation (36a) s'étendant dans la direction de l'extrémité pour la tête de la partie supérieure (31), à l'endroit le plus haut du dispositif de fixation en arc (36), ou dans lequel le dispositif de manipulation (1) est fixé sur un prolongement de fixation (36a) s'étendant dans la direction de l'extrémité pour la tête de la partie supérieure (31), monté latéralement par rapport à l'endroit le plus haut du dispositif de fixation en arc (36) sur le côté opposé au côté des articulations ou charnières (23).

6. Appareil de bronzage (10) selon l'une des revendications 1 à 5, dans lequel le dispositif de manipulation (1) comprend, en tant que dispositifs (4) de sélection et de commande de fonctions dans un appareil de bronzage (10), une touche de navigation multifonction (4a) à commande électronique qui permet d'appeler différents programmes qui sélectionnent et commandent toutes les fonctions de l'appareil de bronzage (10) et d'activer, de modifier ou de désactiver des fonctions individuelles, plusieurs fonctions ou toutes les fonctions des programmes par le biais d'étapes de navigation.

7. Appareil de bronzage (10) selon l'une des revendications 1 à 6, dans lequel le dispositif de manipulation (1) comprend également un dispositif pour stopper toutes les fonctions de l'appareil de bronzage et/ou dans lequel le dispositif de manipulation (1) intègre une ou plusieurs fonction/s de l'appareil de bronzage.

8. Appareil de bronzage (10) selon la revendication 7, dans lequel le dispositif de manipulation (1) comprend, en plus, au moins une buse intégrée pour l'éjection d'une pulvérisation d'une matière dans le compartiment de bronzage (32).

9. Appareil de bronzage (10) selon l'une des revendications 6 à 8, dans lequel le dispositif de manipulation (1) comprend, en plus, au moins un dispositif pour la mise en route d'une installation multimédia intégrée dans l'appareil de bronzage (10).

10. Appareil de bronzage (10) selon la revendication 9, dans lequel le dispositif de manipulation (1) est adapté pour faire en sorte qu'après l'actionnement du dispositif de mise en route d'une installation multimédia intégrée dans l'appareil de bronzage (10), la touche de navigation multifonction (4a) du dispositif de manipulation (1) permette la navigation dans l'installation multimédia.

11. Appareil de bronzage selon l'une des revendications 2 à 10, dans lequel le dispositif de fixation en arc (36) situé dans la zone de la surface de protection (34) de la partie supérieure (31) est pourvu, en plus, d'un affichage ou de plusieurs affichages (37a, 37b, 37c) permettant la reconnaissance de la fonction ou des fonctions considérées au moyen de la touche de navigation multifonction (4a) et qu'il convient de sélectionner.

12. Appareil de bronzage (10) selon la revendication 11, dans lequel l'affichage ou les affichages est/sont un pictogramme ou des pictogrammes qui est/sont un/des pictogramme/s rétro-éclairé/s par des LED, dans lequel le rétro-éclairage par LED du pictogramme/des pictogrammes est choisi de manière à assurer la possibilité de reconnaissance du pictogramme activé/des pictogrammes activés aussi bien lorsque l'appareil de bronzage (10) ne fonctionne pas encore que lorsqu'il fonctionne.

13. Appareil de bronzage (10) selon la revendication 6, comprenant, en plus, un écran d'affichage (37d) de préférence à plusieurs chiffes pour permettre la lecture par l'utilisateur de l'information concernant les paramètres actuellement choisis au moyen de la touche de navigation multifonction (4a).

14. Appareil de bronzage (10) selon l'une des revendications 11 à 13, dans lequel l'affichage individuel ou les plusieurs affichages est/sont situé/s au-dessus de la surface de protection (34).

15. Appareil de bronzage (10) selon l'une des revendications 2 à 14, dans lequel le dispositif de fixation en arc (36) situé dans la zone de la surface de protection (34) de la partie supérieure (31) est pourvu, en plus, d'un affichage ou de plusieurs affichages (38a, 38b) à des fins non liées à des fonctions.

16. Partie supérieure pour un appareil de bronzage, qui peut soit être montée sur un des côtés longitudinaux (22) de la partie inférieure (21) d'un appareil de bronzage (10) par le biais de charnières ou d'articulations (23) et peut, en basculant ou en pivotant par le biais des charnières ou articulations (23), être éloignée de la partie inférieure (21) et rabattue sur la partie inférieure (21) pour former un compartiment de bronzage (32), soit être éloignée de la partie inférieure (21) d'un appareil de bronzage (10) par élévation et abaissée sur la partie inférieure (21), pour former un compartiment de bronzage (32), sur une suspension séparée, la partie supérieure (31) comprenant une extrémité pour la tête, une extrémité pour les pieds, deux côtés longitudinaux et un dispositif de manipulation (1) en vue d'une manipulation manuelle, y compris la fermeture et l'ouverture, de la partie supérieure (31), le dispositif de manipulation (1) pouvant être installé sur la partie supérieure (31) de l'appareil de bronzage (10) à un endroit que l'utilisateur peut atteindre et comprenant des dispositifs (4) pour sélectionner et commander au moins les fonctions Mise en marche de l'appareil de bronzage (10) et Déroulement du processus de bronzage de l'appareil de bronzage (10).

17. Partie supérieure (31) pour un appareil de bronzage (10) comprenant un dispositif de manipulation (1) selon la revendication 16, qui peut être installé sur la partie supérieure (31) de l'appareil de bronzage (10) dans la zone de la surface de protection (34), sur un dispositif de fixation en arc (36) pourvu d'un prolongement de fixation (36a) s'étendant dans la direction de l'extrémité pour la tête.

18. Partie supérieure (31) pour un appareil de bronzage (10) comprenant un dispositif de manipulation (1) selon la revendication 16 ou la revendication 17, le dispositif de manipulation (1) ayant la forme d'une poignée en U (2a) ou d'une poignée ouverte (2b) ou d'une poignée similaire à une barre (2c) ou d'une poignée similaire à un bouton de porte (2d) ou d'une poignée en creux encastrée (2e) dans la surface de protection (34).

19. Partie supérieure (31) pour un appareil de bronzage (10) comprenant un dispositif de manipulation (1) selon la revendication 18, le dispositif de manipulation (1) ayant la forme d'une poignée ouverte (2b) disposée dans la direction longitudinale du compartiment de bronzage (32) avec son extrémité ouverte dirigée vers l'extrémité pour les pieds du compartiment de bronzage (32).

20. Partie supérieure (31) pour un appareil de bronzage (10) comprenant un dispositif de manipulation (1) selon la revendication 16, 17 ou 18, le dispositif de manipulation (1) étant fixé sur le prolongement de fixation (36a) s'étendant dans la direction de l'extrémité pour la tête de la partie supérieure (31), à l'endroit le plus haut du dispositif de fixation en arc (36), ou étant fixé sur le prolongement de fixation (36a) s'étendant dans la direction de l'extrémité pour la tête de la partie supérieure (31), monté latéralement par rapport à l'endroit le plus haut du dispositif de fixation en arc (36) sur le côté opposé au côté des articulations ou charnières (23).

21. Partie supérieure (31) pour un appareil de bronzage (10) comprenant un dispositif de manipulation (1) selon l'une des revendications 16 à 20, le dispositif de manipulation (1) comprenant, en tant que dispositifs (4) de sélection et de commande de fonctions dans un appareil de bronzage, une touche de navigation multifonction (4a) à commande électronique qui permet d'appeler différents programmes qui sélectionnent et commandent toutes les fonctions de l'appareil de bronzage et d'activer, de modifier ou de désactiver des fonctions individuelles, plusieurs fonctions ou toutes les fonctions des programmes par le biais d'étapes de navigation.

22. Partie supérieure (31) pour un appareil de bronzage (10) comprenant un dispositif de manipulation (1) selon l'une des revendications 16 à 21, le dispositif de manipulation (1) comprenant également un dispositif pour stopper toutes les fonctions de l'appareil de bronzage (10).

23. Partie supérieure (31) pour un appareil de bronzage (10) comprenant un dispositif de manipulation (1) selon l'une des revendications 16 à 22, le dispositif de manipulation (1) intégrant, en plus, une ou plusieurs fonction/s de l'appareil de bronzage.

24. Partie supérieure (31) pour un appareil de bronzage (10) comprenant un dispositif de manipulation (1) selon la revendication 23, le dispositif de manipulation (1) comprenant, en plus, une buse intégrée pour l'éjection d'une pulvérisation aqueuse dans la zone pour la tête du compartiment de bronzage (32).

25. Partie supérieure (31) pour un appareil de bronzage (10) comprenant un dispositif de manipulation (1) selon la revendication 23, le dispositif de manipulation (1) comprenant, en plus, au moins un dispositif pour la mise en route d'une installation multimédia intégrée dans l'appareil de bronzage.

26. Partie supérieure (31) pour un appareil de bronzage (10) comprenant un dispositif de manipulation (1) selon la revendication 25, dans lequel, après l'actionnement du dispositif de mise en route d'une installation multimédia intégrée dans l'appareil de bronzage, la touche de navigation multifonction (4a) du dispositif de poignée (1) permet la navigation dans l'installation multimédia.
